# EUROPEAN PATENT APPLICATION

(11) **EP 4 772 650 A1**
(43) Date of publication of application: **08.07.2026**
(21) Application number: 25802435.5
(22) Date of filing: 18.04.2025
(51) Int. Cl.: C12Q 1/68

(54) **PRIMER/PROBE COMPOSITION FOR CAPILLARY ELECTROPHORESIS ANALYSIS, DETECTION METHOD, AND USE**

(30) Priority: 17.05.2024 CN 202410614667
(71) Applicant: Ningbo Health Gene Technologies Co., Ltd., Ningbo, Zhejiang 315040 (CN)
(72) Inventor: GONG, Yangqing, Ningbo, Zhejiang 315302 (CN); WU, Yong, IRVine California 92612 (US)
(74) Representative: Lock, Graham James
(86) International application number: PCT/CN2025/089715
(87) International publication number: WO 2025/236986

(57) **Abstract**

The present invention provides a primer/probe composition for capillary electrophoresis analysis, a detection method, and a use. The primer/probe composition comprises: a forward primer, a reverse primer, and a nucleic acid probe. The 5' end of the nucleic acid probe carries a fluorescent label, and neither the forward primer nor the reverse primer carries a fluorescent label.

## Description

### CROSS-REFERENCE TO RELATED APPLICATIONS

This application claims the benefit of Chinese patent application No. CN202410614667.4, filed on May 17, 2024, which is hereby incorporated by reference in its entirety.

### TECHINICAL FIELD

The present invention belongs to the field of biochemistry, and particularly relates to a primer/probe composition for capillary electrophoresis analysis, detection methods and uses thereof.

### BACKGROUND OF THE INVENTION

Capillary electrophoresis (CE) detection technology is a technology in which nucleic acids are sequentially arranged from small to large based on fragment lengths by means of electrophoresis, so as to detect a target, and a specific process thereof generally comprises: 1. designing and synthesizing primers with fluorescent labels; 2. amplification by polymerase chain reaction (PCR) to obtain nucleic acids with different lengths of fluorescent labels; 3. separating the amplification products by capillary electrophoresis; and 4 determining whether the target nucleic acid is present in the detected samples according to the fluorescent position and intensity of the amplification products.

At present, the disadvantages of existing capillary electrophoresis detection technologies include: 1. different detection targets need to pass through design primers, so that the distance between the forward primer and the reverse primer on the amplification target is different, so as to obtain fragments of different lengths; 2, the PCR amplification efficiency of amplification products of different lengths decreases with the increase of the length; 3, since there are a large number of different primers in the same reaction system that are prone to non-specific amplification, the non-specific amplification products generated by the fluorescent labeled primer easily interfere with the target fluorescent signal; and 4, the high-concentration sample easily generates the supersaturated fluorescent signal to cause the baseline to rise, thereby affecting the determination of the adjacent target point.

### SUMMARY OF THE INVENTION

The purpose of the present invention is to overcome the defects in the prior art, and to provide a primer/probe composition for capillary electrophoresis analysis, a detection method and an application thereof. In particular, the primer/probe compositions of the present invention can be used for nucleic acid detection of single and multiple targets.

Before describing the content of the present invention, the terms used herein are defined as follows.

The term "forward primer", also referred to as upstream primer, means the upstream junction position along the positive strand (sense strand, coding strand) in double-stranded DNA.

The term "reverse primer", also referred to as downstream primer, means the downstream junction position along the negative strand (complementary strand) in double-stranded DNA.

The term "nt" refers to a nucleotide, which is a class of compounds consisting of 1) purine bases or pyrimidine bases, 2) ribose or deoxyribose, and 3) phosphoric acid.

The term "PCR" refers to a polymerase chain reaction.

The term "CE" refers to capillary electrophoresis.

The term "dNTP" refers to deoxy-ribonucleoside triphosphates.

The term "DNA polymerase" refers to an enzyme that catalyzes the polymerization of substrate dNTP molecules to form progeny DNAs by utilizing parent DNAs as templates.

The term "nucleic acid probe" refers to a class of nucleotides that consist of a single or a mixture of ribonucleotides and deoxyribonucleotides.

The term "nucleotide length difference" refers to the difference in length between any nucleic acid probe and the nearest nucleic acid probe.

The term "conventional DNA" refers to deoxyribonucleic acids formed by four deoxyribonucleotides (i.e. adenine deoxyribonucleotides, guanine deoxyribonucleotides, cytosine deoxyribonucleotides, thymine deoxyribonucleotides) as basic component units.

The term "conventional RNA" refers to a ribonucleic acid formed by four ribonucleotides (i.e. adenine ribonucleotides, guanine ribonucleotides, cytosine ribonucleotides, uracil ribonucleotides) as basic component units.

The term "DNA probe" refers to a DNA fragment with a label.

The term "RNA probe" refers to an RNA fragment with a label.

The term "DNA/RNA chimeric probe" refers to a fragment consisting of labeled deoxyribonucleotides and ribonucleotides.

The term "non-complementary nucleotide" refers to a nucleotide chain that cannot form a stable hydrogen bond with a template strand.

The term "ddNTP" refers to dideoxyribonucleoside triphosphates, including ddATP, ddTTP, ddGTP, ddCTP.

The term "Spacer modification" refers to a meta-arm modification consisting of straight carbon chain(s) or ethylene glycol(s), including Spacer C3, Spacer C6, Spacer C9, etc.

The term "TE buffer" refers to a liquid reagent that consists of Tris and EDTA, in capable of stabilizing nucleic acids and resisting pH changes when added to a small amount of acid/base.

The term "locked nucleic acid" refers to an oligonucleotide derivative, which belongs to a bicyclic nucleotide derivative and has a similar phosphoric acid skeleton to DNA and RNA. And the 2' and 4' ends of locked nucleic acid ribose form an oxymethylene bridge, a sulfur methylene bridge or an amine methylene bridge.

The term "hypoxanthine" refers to 6-hydroxypurine, is represented by capital letter "I", and can be complementary to adenine, cytosine and uracil.

The term "Alexa Fluor 405" refers to one of the Alexa Fluor series of fluorescent dyes, with a maximum excitation wavelength of 401 nm and a maximum emission wavelength of 421 nm. Representative manufacturers include, but are not limited to, Thermo Fisher Scientific, Molecular Probes, Inc.

The term "Alexa Fluor 350" refers to one of the Alexa Fluor series of fluorescent dyes, with a maximum excitation wavelength of 346 nm and a maximum emission wavelength of 442 nm. Representative manufacturers include, but are not limited to, Thermo Fisher Scientific, Molecular Probes, Inc.

The term "FAM" refers to a fluorescent dye including a structure of 5 (6)-carboxyfluorescein (5-(and -6)-Carboxyfluorescein, molecular formula: C₂₁H₁₂O₇), with a maximum excitation wavelength of 493-494 nm and a maximum emission wavelength of 518 nm. Representative manufacturers include, but are not limited to, Bioengineering (Shanghai) Co. Ltd. and Thermo Fisher Scientific Corporation.

The term "Alexa Fluor 488" refers to one of the Alexa Fluor series of fluorescent dyes, with a maximum excitation wavelength of 496 nm and a maximum emission wavelength of 519 nm. Representative manufacturers include, but are not limited to, Thermo Fisher Scientific, Molecular Probes, Inc.

The term "Atto 488" refers to a fluorescent dye having a maximum excitation wavelength of 499 nm and a maximum emission wavelength of 520 nm. Representative manufacturers include, but are not limited to, the United States AAT Bioquest Company.

The term "TET" refers to a fluorescent dye including a structure of (4,6-dichlorotriazinyl) amino fluorescein (molecular formula: C₂₁H₈C₁₄O₇), with a maximum excitation wavelength of 521 nm and a maximum emission wavelength of 536-542 nm. Representative manufacturers include, but are not limited to, Shenggong bioengineering (Shanghai) Co. Ltd. and Thermo Fisher Scientific Corporation.

The term "VIC" refers to a fluorescent dye with a maximum excitation wavelength of 525-538 nm and a maximum emission wavelength of 545-554 nm. Representative manufacturers include, but are not limited to, Shenggong bioengineering (Shanghai) Co. Ltd. and Thermo Fisher Scientific Corporation.

The term "R6G" refers to a fluorescent dye including a structure of rhodamine 6G (molecular formula: C₂₈H₃₁ClN₂O₃), with a maximum excitation wavelength of 522-530 nm and a maximum emission wavelength of 546-548 nm. Representative manufacturers include, but are not limited to, Shenggong bioengineering (Shanghai) Co. Ltd.

The term "JOE" refers to a fluorescent dye including a structure of 4', 5'-dichloro -2', 7'-dimethoxyfluorescein (molecular formula: C₂₃H₁₄Cl₂O₉), with a maximum excitation wavelength of 520 nm, and a maximum emission wavelength of 548 nm. Representative manufacturers include, but are not limited to, Shenggong bioengineering (Shanghai) Co. Ltd. and Thermo Fisher Scientific Corporation.

The term "HEX" refers to a fluorescent dye, which main components including a structure of 2',4,4',5',7,7'-hexachlorfluorescein (molecular formula: C₂₅H₉Cl₆NO₉), with a maximum excitation wavelength of 533-535 nm and a maximum emission wavelength of 550-556 nm. Representative manufacturers include, but are not limited to, Shenggong bioengineering (Shanghai) Co. Ltd. and Thermo Fisher Scientific Corporation.

The term "Alexa Fluor 532" refers to one of the Alexa Fluor series of fluorescent dyes, with a maximum excitation wavelength of 532 nm and a maximum emission wavelength of 553 nm. Representative manufacturers include, but are not limited to, Thermo Fisher Scientific, Molecular Probes, Inc.

The term "Cy3" refers to anthocyanin fluorescent dye Cy3 (molecular formula: C₃₀H₃₇ClN₂O₂), with a maximum excitation wavelength of 552-555 nm and a maximum emission wavelength of 565-570 nm. Representative manufacturers include, but are not limited to, Shenggong bioengineering (Shanghai) Co. Ltd. and Thermo Fisher Scientific Corporation.

The term "Alexa Fluor 555" refers to one of the Alexa Fluor series of fluorescent dyes, with a maximum excitation wavelength of 555 nm and a maximum emission wavelength of 565 nm. Representative manufacturers include, but are not limited to, Thermo Fisher Scientific, Molecular Probes, Inc.

The term "NED" refers to a fluorescent dye having a maximum excitation wavelength of 545-546 nm and a maximum emission wavelength of 567-575 nm. Representative manufacturers include, but are not limited to, Shenggong bioengineering (Shanghai) Co. Ltd. and Thermo Fisher Scientific Corporation.

The term "Alexa Fluor 546" refers to one of the Alexa Fluor series of fluorescent dyes having a maximum excitation wavelength is 556 nm and a maximum emission wavelength is 573 nm. Representative manufacturers include, but are not limited to, Thermo Fisher Scientific, Molecular Probes, Inc.

The term "TAMRA" refers to a fluorescent dye having a main component of 5-carboxytetramethylrhodamine (molecular formula: C₃₁H₂₈N₄O₆), with a maximum excitation wavelength of 552-565 nm and a maximum emission wavelength of 578-580 nm. Representative manufacturers include, but are not limited to, Shenggong bioengineering (Shanghai) Co. Ltd. and Thermo Fisher Scientific Corporation.

The term "ROX" refers to a fluorescent dye having a main component of X-rhodamine (molecular formula: C₃₉H₃₆N₄O₆), with a maximum excitation wavelength of 578-585 nm and a maximum emission wavelength of 602-605 nm. Representative manufacturers include, but are not limited to, Shenggong bioengineering (Shanghai) Co. Ltd. and Thermo Fisher Scientific Corporation.

The term "Texas Red" refers to a fluorescent dye with a maximum excitation wavelength of 586-595 nm and a maximum emission wavelength of 603-615 nm. Representative manufacturers include, but are not limited to, Shenggong bioengineering (Shanghai) Co. Ltd. and AAT Bioquest in the United States.

The term "Alexa Fluor 568" refers to one of the Alexa Fluor series of fluorescent dyes, with a maximum excitation wavelength of 578 nm and a maximum emission wavelength of 603 nm. Representative manufacturers include, but are not limited to, Thermo Fisher Scientific, Molecular Probes, Inc.

The term "Alexa Fluor 594" refers to one of the Alexa Fluor series of fluorescent dyes, with a maximum excitation wavelength of 590 nm and a maximum emission wavelength of 617 nm. Representative manufacturers include, but are not limited to, Thermo Fisher Scientific, Molecular Probes, Inc.

The term "Alexa Fluor 633" refers to one of the Alexa Fluor series of fluorescent dyes, with a maximum excitation wavelength of 632 nm and a maximum emission wavelength of 647 nm. Representative manufacturers include, but are not limited to, Thermo Fisher Scientific, Molecular Probes, Inc.

The term "Alexa Fluor 647" refers to one of the Alexa Fluor series of fluorescent dyes, with a maximum excitation wavelength of 650 nm and a maximum emission wavelength of 665 nm. Representative manufacturers include, but are not limited to, Thermo Fisher Scientific, Molecular Probes, Inc.

The term "Cy5" refers to an anthocyanin fluorescent dye Cy5 (molecular formula: C₃₃H₃₉KN₂O₈S₂), with a maximum excitation wavelength of 643-651 nm and a maximum emission wavelength of 667-670 nm. Representative manufacturers include, but are not limited to, Shenggong bioengineering (Shanghai) Co. Ltd. and Thermo Fisher Scientific Corporation.

The term "SIZE" refers to a standard substance for determining an electrophoretic migration location.

The term "Sanger sequencer" refers to a gene sequencer on the basis of dideoxy chain termination sequencing methods.

The term "P" refers to a phosphorylation label.

The first aspect of the present invention provides a primer/probe composition for capillary electrophoresis analysis, the primer/probe composition comprising:
a forward primer,
a reverse primer, and
a nucleic acid probe carrying a fluorescent label;
wherein neither the forward primer nor the reverse primer carries a fluorescent label, and the fluorescent label is preferably located at the 5'end of the nucleic acid probe.

The primer/probe composition according to the first aspect of the invention, wherein:
the nucleic acid probe is a probe group with a nucleotide length difference of 1-100, and the nucleotide length difference of each nucleic acid probe in the probe group preferably is 1-50, more preferably is 1-20, and further preferably is 1-10;
the number of nucleotides of the forward primer sequence is 15-60, preferably is 18-40, more preferably is 18-30, and further preferably is 20-25;
the number of nucleotides of the reverse primer sequence is 15-60, preferably is 18-40, more preferably is 19-31, and further preferably is 19-26; and/or
the fluorescent label is one or more selected from the following: a fluorescent label with a maximum fluorescence emission wavelength of 400-525 nm, a fluorescent label with a maximum fluorescence emission wavelength of 526-560 nm, a fluorescent label with a maximum fluorescence emission wavelength of 561-580 nm, a fluorescent label with a maximum fluorescence emission wavelength of 581-620 nm, and a fluorescent label with a maximum fluorescence emission wavelength of 621-670 nm.

The primer/probe composition according to the first aspect of the invention, wherein:
the fluorescent label with the maximum fluorescence emission wavelength at 400-525 nm is one or more selected from Alexa Fluor 405, Alexa Fluor 350, FAM, Alexa Fluor 488, and Atto 488, preferably is FAM or Alexa Fluor 488, most preferably is FAM;
the fluorescent label with the maximum fluorescence emission wavelength of 526-560 nm is one or more selected from JOE, TET, R6 G, VIC, HEX, and Alexa Fluor 532, preferably is one or more selected from JOE, VIC, and TET, more preferably is JOE or VIC;
the fluorescent label with the maximum fluorescence emission wavelength of 561-580 nm is one or more selected from Cy3, Alexa Fluor 555, NED, Alexa Fluor 546, and TAMRA, preferably is Cy3 or NED, and most preferably is Cy3;
the fluorescent label with the maximum fluorescence emission wavelength in 581-620 nm is one or more selected from ROX, Texas Red, Alexa Fluor 568, and Alexa Fluor 594, preferably is ROX or Texas Red, most preferably is ROX; and/or
the fluorescent label with the maximum fluorescence emission wavelength of 621-670 nm is one or more selected from Cy5, Alexa Fluor 633, and Alexa Fluor 647, preferably is Cy5 or Alexa Fluor 647, most preferably is Cy5.

The primer/probe composition according to the first aspect of the present invention, wherein the nucleic acid probe is one or more selected from the following: a DNA probe, an RNA probe, a DNA/RNA chimeric probe, preferably is a DNA probe or an RNA probe, most preferably is a DNA probe; wherein:
the DNA probe is one or more selected from the following: a conventional DNA probe, a DNA probe comprising a lock nucleic acid, and a DNA probe comprising hypoxanthine, most preferably is a conventional DNA probe; and/or
the RNA probe is one or more selected from of the following: a conventional RNA probe, an RNA probe comprising a lock nucleic acid, and an RNA probe comprising hypoxanthine, most preferably is a conventional RNA probe.

The primer/probe composition according to the first aspect of the present invention, wherein the 3' end of the nucleic acid probe further carries a label for inhibiting extension of the nucleic acid probe;
preferably, the label for inhibiting extension of the nucleic acid probe is one or more selected from the following: a fluorescent label, a phosphoric acid group, a non-complementary nucleotide, a ddNTP, and a Spacer modification, more preferably is one or more selected from the following: a phosphoric acid group, a non-complementary nucleotide, and a ddNTP, further preferably is a phosphoric acid group or a ddNTP.

The second aspect of the present invention provides an improved capillary electrophoresis detection method, comprising the following steps:
1) preparing a reaction system using the primer/probe composition for capillary electrophoresis analysis described in the first aspect;
2) amplifying the reaction system prepared in step 1) by PCR reaction to obtain an amplification product;
3 ) separating the product amplified in step 2) by capillary electrophoresis.

The capillary electrophoresis detection method according to the second aspect of the present invention, wherein the step 1) comprises:
a) designing, synthesizing a forward primer and a reverse primer, and fluorescent labeling the nucleic acid probe;
b) preparing the synthesized forward primer and reverse primer, the fluorescent labeled nucleic acid probe, a reaction buffer, a DNA polymerase, dNTP, a sample to be tested, and a negative sample into a reaction system.

The capillary electrophoresis detection method according to the second aspect of the present invention, wherein in step b):
the negative sample is one or more selected from the following: purified water, a TE buffer, a plasmid sample that does not contain a target nucleic acid, a microbial isolate, and a tissue sample, preferably is one or more selected from the following: purified water, a TE buffer, a plasmid sample that does not contain a target nucleic acid, and a microbial isolate, more preferably is one or more selected from: purified water, a TE buffer, and a plasmid sample that does not contain a target nucleic acid; and/or
the reaction buffer is a Tris-hydrochloric acid buffer or a PBS buffer; and the reaction buffer preferably comprises a cation, and the cation is preferably one or more selected from magnesium ions, potassium ions, sodium ions, and calcium ions, more preferably is magnesium ions or potassium ions.

The capillary electrophoresis detection method according to the second aspect of the present invention, wherein in the reaction system formulated in step b):
the forward primer has a concentration of 50 nM -5000 nM, preferably 100 nM -2000 nM, more preferably 100 nM -300 nM;
the concentration of the reverse primer is 50 nM -5000 nM, preferably 100 nM -2000 nM, more preferably 100 nM -300 nM;
the concentration of the nucleic acid probe is 5 nM -500 nM, preferably 5 nM -200 nM, more preferably 5 nM -15 nM.

The capillary electrophoresis detection method according to the second aspect of the present invention, wherein in step a):
the operation of fluorescent labeling the nucleic acid probe further includes one or more of the following:
(1) fluorescent labeling the 5' end of the nucleic acid probe while modifying the 3' end of the nucleic acid probe,
(2) fluorescent labeling the 5' end of the nucleic acid probe while making the 3' end base of the nucleic acid probe not completely complementary to the template, and
(3) fluorescent labeling the 3' end of the nucleic acid probe.

The capillary electrophoresis detection method according to the second aspect of the present invention, wherein the improved capillary electrophoresis detection method further comprises:
4) analyzing the target nucleic acid in the sample to be tested by comparing the fluorescent signal intensity of the nucleic acid probe.

The third aspect of the present invention provides a use of the primer/probe composition according to the first aspect in the preparation of a reagent product and/or a detection device for capillary electrophoresis analysis, wherein:
the reagent product is preferably a kit; and/or
the detection device is preferably a capillary electrophoresis instrument or a DNA sequencer;
preferably, the DNA sequencer is a Sanger sequencer.

The fourth aspect of the present invention provides a reagent product for capillary electrophoresis analysis, wherein the reagent product comprises the primer/probe composition according to the first aspect;
preferably, the reagent product is a kit.

The fifth aspect of the present invention provides a detection device for capillary electrophoresis analysis, wherein the detection device comprises the primer/probe composition according to the first aspect.

The detection device according to the fifth aspect of the present invention, wherein the detection device is a capillary electrophoresis instrument or a DNA sequencer; preferably, the DNA sequencer is a Sanger sequencer.

According to a specific embodiment of the present invention, the primer used in the present invention comprises a forward primer, a reverse primer, and a nucleic acid probe. The forward primer does not have a fluorescent label, but the reverse primer does not have a fluorescent label. The fluorescent label is added to the 5' end of the nucleic acid probe, and the addition of 3' end label inhibits the extension of the nucleic acid probe.

According to the above specific embodiment of the present invention, the operation steps of the present invention are as follows:
Firstly, a suitable reaction system is selected, and a reaction buffer (including DNA polymerase, dNTP), a forward primer, a reverse primer, a nucleic acid probe, a sample to be tested or a negative sample is added to prepare a suitable reaction system.

Secondly, a suitable reaction procedure is selected for PCR amplification.

Finally, the PCR product was subjected to electrophoresis to determine whether the target nucleic acid was positive by comparing the fluorescent signal intensity of the target nucleic acid probe and the non-target nucleic acid probe.

The technical problems to be solved by the present invention are as follows:
1. In the prior art at present, capillary electrophoresis (CE) detection technology distinguishes different targets by separating different amplification product lengths. The present invention recognizes different targets by detecting nucleic acid probes, so that the target recognition is not limited by the length of the amplification products.
2. In the prior art at present, capillary electrophoresis (CE) detection technology uses amplification primers to carry fluorescent labels, and when the primers have non-specific amplification, their non-specific amplification products will affect the interpretation of the target signal. In the amplification system of the present invention, only the nucleic acid probe carries the fluorescent signal, and no additional signal is generated.
3. In the prior art at present, capillary electrophoresis (CE) detection technology uses an excessive primer, and when the concentration of a target nucleic acid sequence in a sample is too high, a saturated complex peak is generated, and the interference is determined adjacent to the target point. The present invention can effectively prevent the generation of supersaturated fluorescent signals by using specific concentration nucleic acid probes.

In the prior art, fluorescent labeling is performed at the 5' end of the forward primer, different products with fluorescent labels are generated in the PCR process, and products of different lengths are separated by electrophoresis.

In the primer/probe composition for capillary electrophoresis and the detection method of the present invention, the forward primer is not fluorescently labeled, only the nucleic acid probe carries a fluorescent label, and the nucleic acid probe is cut during the PCR process, and the fluorescent signal of the nucleic acid probe disappears or is weakened in the electrophoresis process.

Compared with the prior art, the primer/probe composition for capillary electrophoresis and the detection method of the present invention can have, but are not limited to, the following beneficial effects:
1 in the present invention, different targets are identified by detecting the nucleic acid probe, so that the target point is not limited by the length of the amplification product;
2 in the amplification system of the present invention, only the nucleic acid probe carries the fluorescent signal and does not generate the non-specific fluorescent signal;
3 the present invention could effectively prevent the generation of supersaturated fluorescent signals by using specific concentration nucleic acid probes;
4 the fluorescent signals between different targets do not interfere with each other, and multi-color fluorescence can be used at the same time.

### BRIEF DESCRIPTION OF THE DRAWINGS

Hereinafter, the embodiments of the present invention will be described in detail with reference to the drawings, in which:

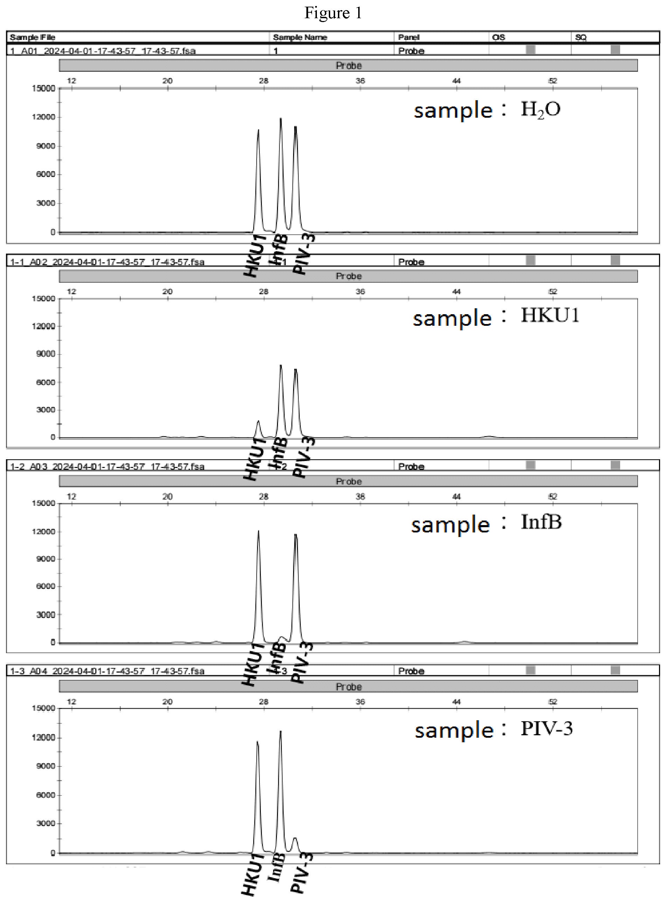

Figure 1 shows the detection effect of the present invention of the primer/probe composition for capillary electrophoresis of the present invention in Example 1, wherein 5' end of the nucleic acid probe carries a FAM fluorescent label, 3' end has a phosphorylation modification, and the length of the nucleic acid probe is 1 nt.

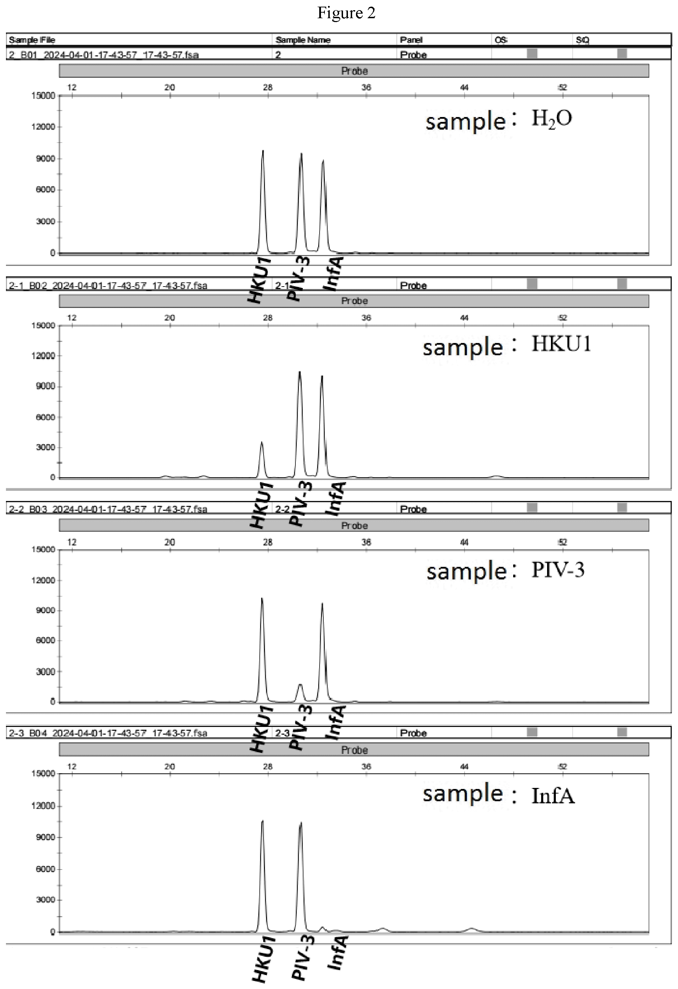

Figure 2 shows the detection effect of the present invention of the primer/probe composition for capillary electrophoresis of the present invention in Example 2, wherein 5' end of the nucleic acid probe carries a FAM fluorescent label, 3' end has a phosphorylation modification, and the length of the nucleic acid probe is 2 nt.

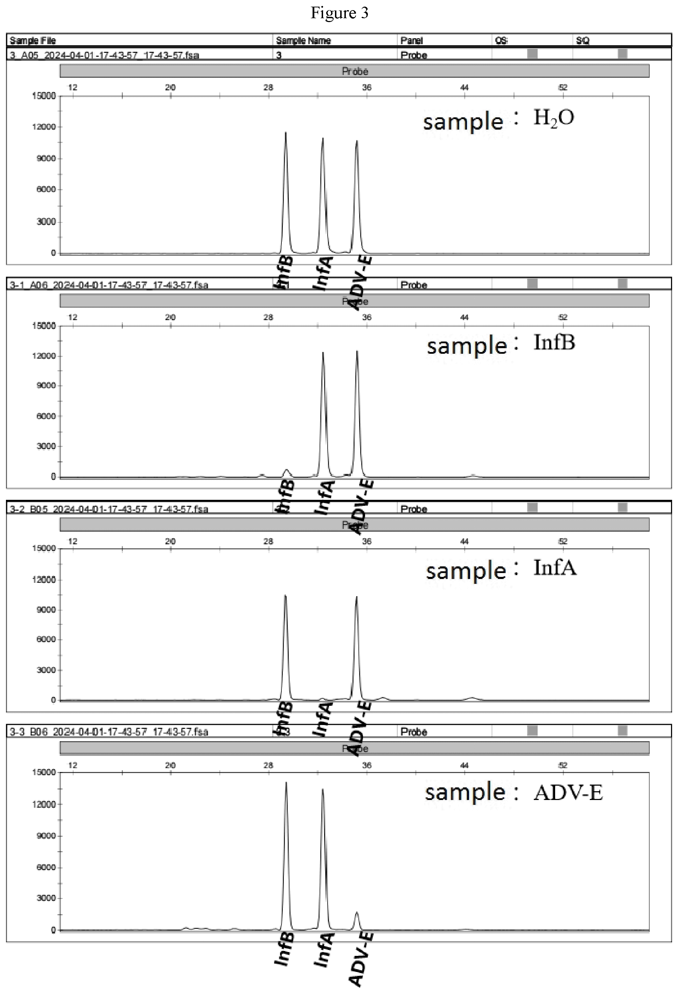

Figure 3 shows the detection effect of the present invention of the primer/probe composition for capillary electrophoresis of the present invention in Example 3, wherein 5' end of the nucleic acid probe carries a FAM fluorescent label, 3' end has a phosphorylation modification, and the length of the nucleic acid probe is 3 nt.

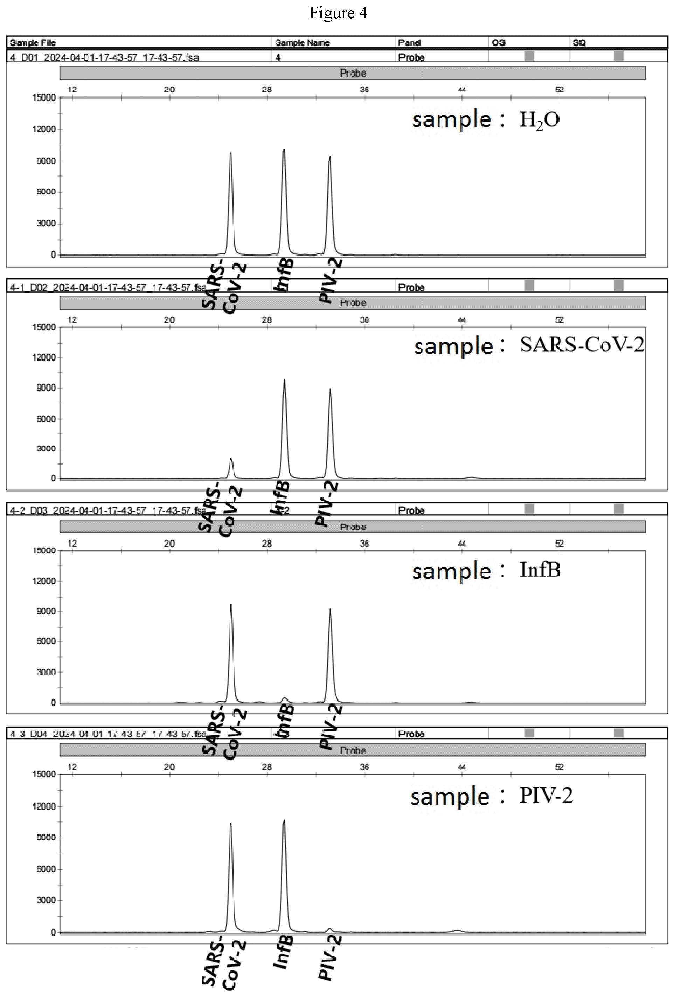

Figure 4 shows the detection effect of the present invention of the primer/probe composition for capillary electrophoresis of the present invention in Example 4, wherein 5' end of the nucleic acid probe carries a FAM fluorescent label, 3' end has a phosphorylation modification, and the length of the nucleic acid probe is 4 nt.

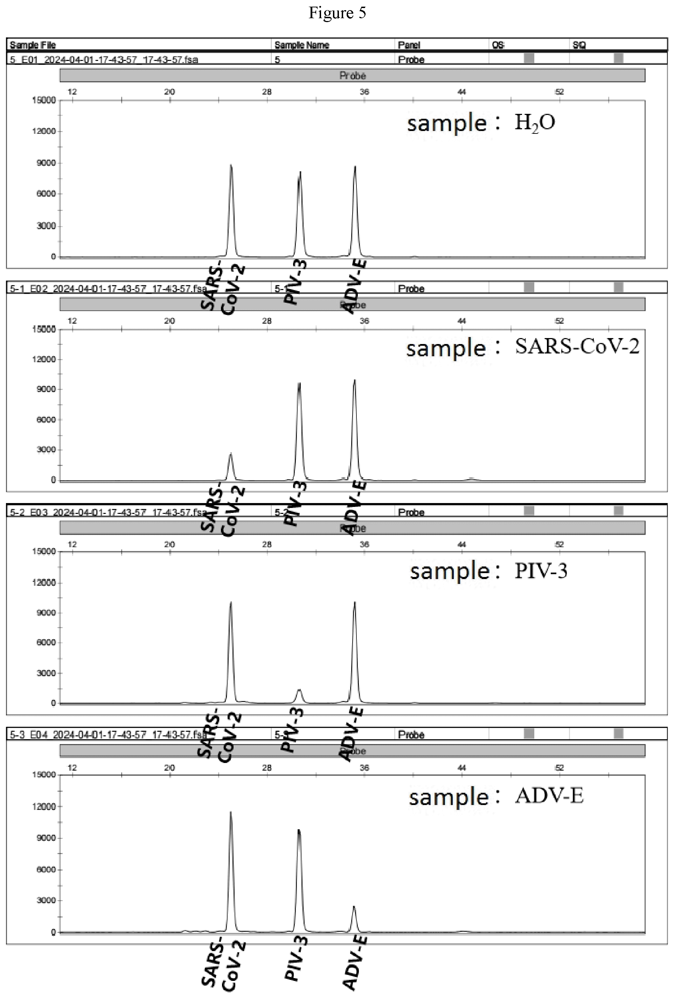

Figure 5 shows the detection effect of the present invention of the primer/probe composition for capillary electrophoresis of the present invention in Example 5, wherein 5' end of the nucleic acid probe carries a FAM fluorescent label, 3' end has a phosphorylation modification, and the length of the nucleic acid probe is 5 nt.

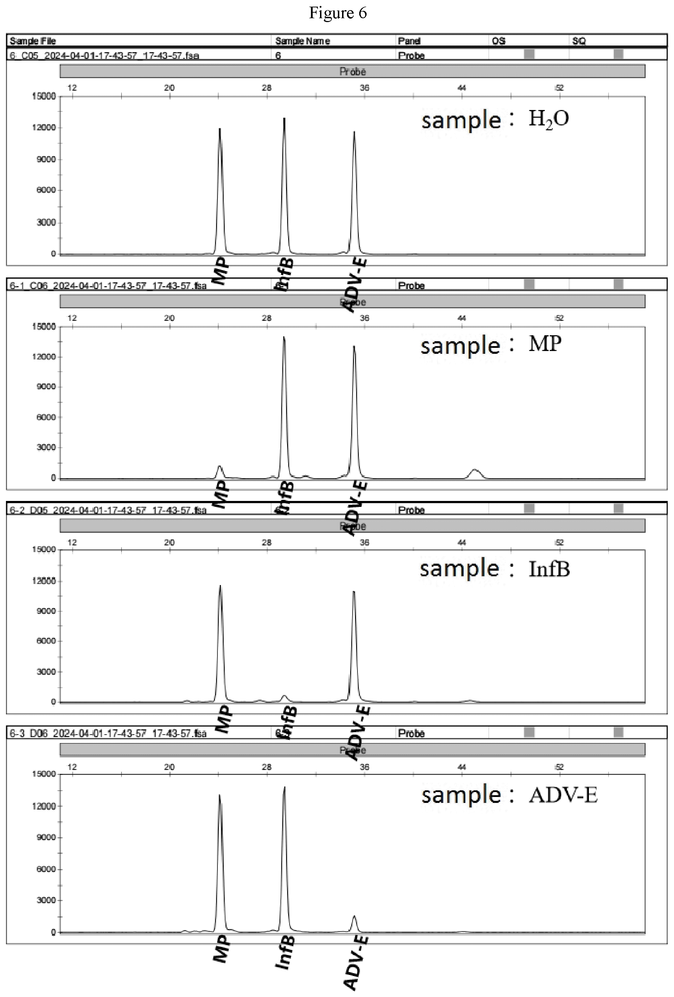

Figure 6 shows the detection effect of the present invention of the primer/probe composition for capillary electrophoresis of the present invention in Example 6, wherein 5' end of the nucleic acid probe carries a FAM fluorescent label, 3' end has a phosphorylation modification, and the length of the nucleic acid probe is 6 nt.

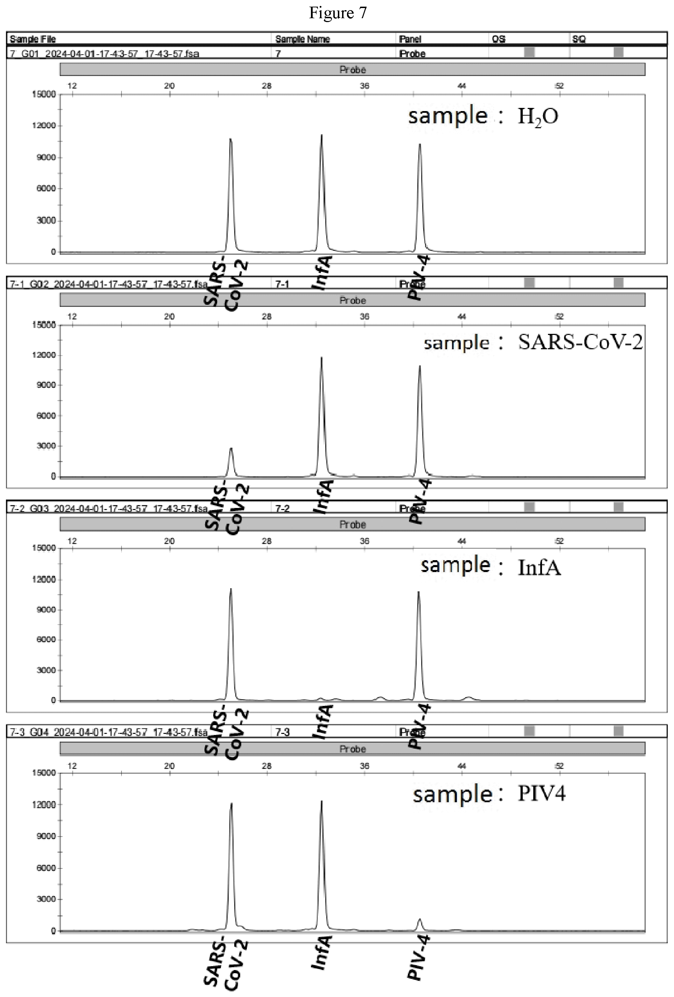

Figure 7 shows the detection effect of the present invention of the primer/probe composition for capillary electrophoresis of the present invention in Example 7, wherein 5' end of the nucleic acid probe carries a FAM fluorescent label, 3' end has a phosphorylation modification, and the length of the nucleic acid probe is 7 nt.

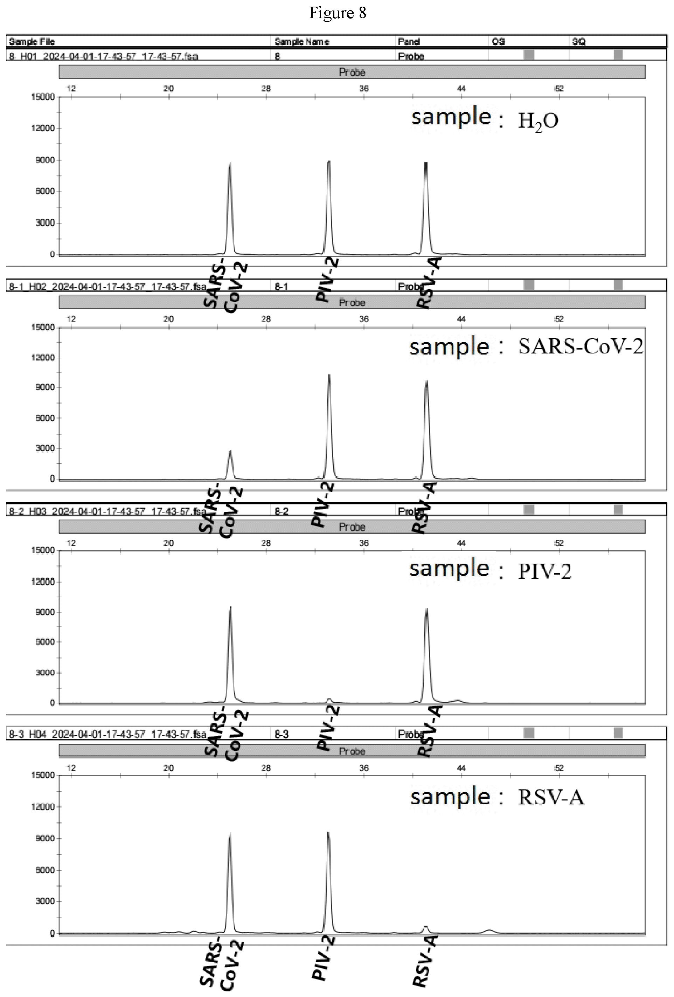

Figure 8 shows the detection effect of the present invention of the primer/probe composition for capillary electrophoresis of the present invention in Example 8, wherein 5' end of the nucleic acid probe carries a FAM fluorescent label, 3' end has a phosphorylation modification, and the length of the nucleic acid probe is 8 nt.

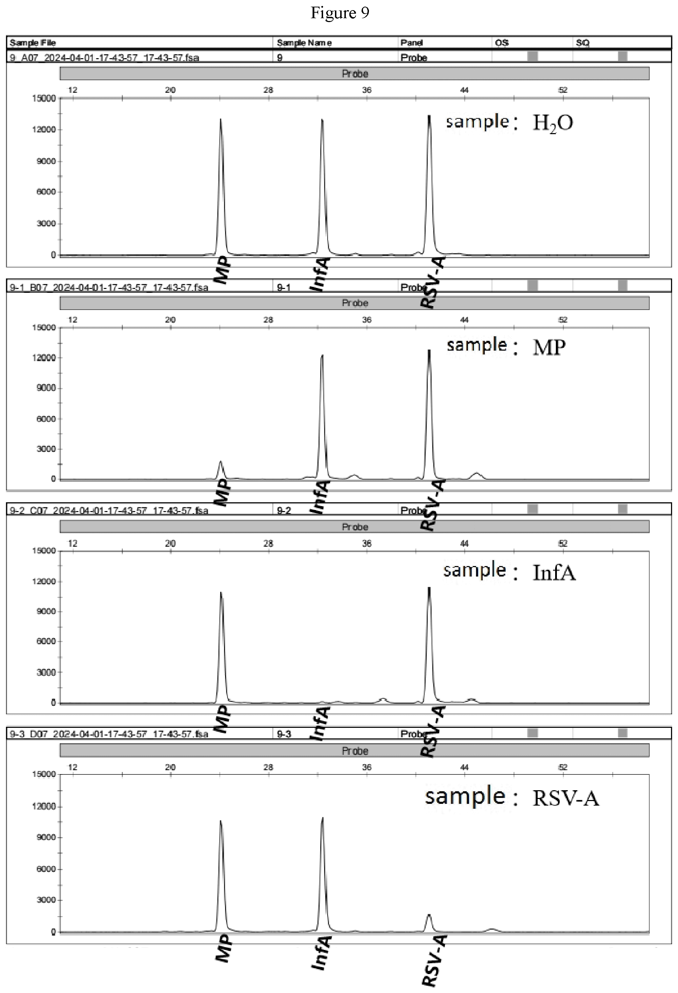

Figure 9 shows the detection effect of the present invention of the primer/probe composition for capillary electrophoresis of the present invention in Example 9, wherein 5' end of the nucleic acid probe carries the FAM fluorescent label, 3' end has a phosphorylation modification, and the length of the nucleic acid probe is 9 nt.

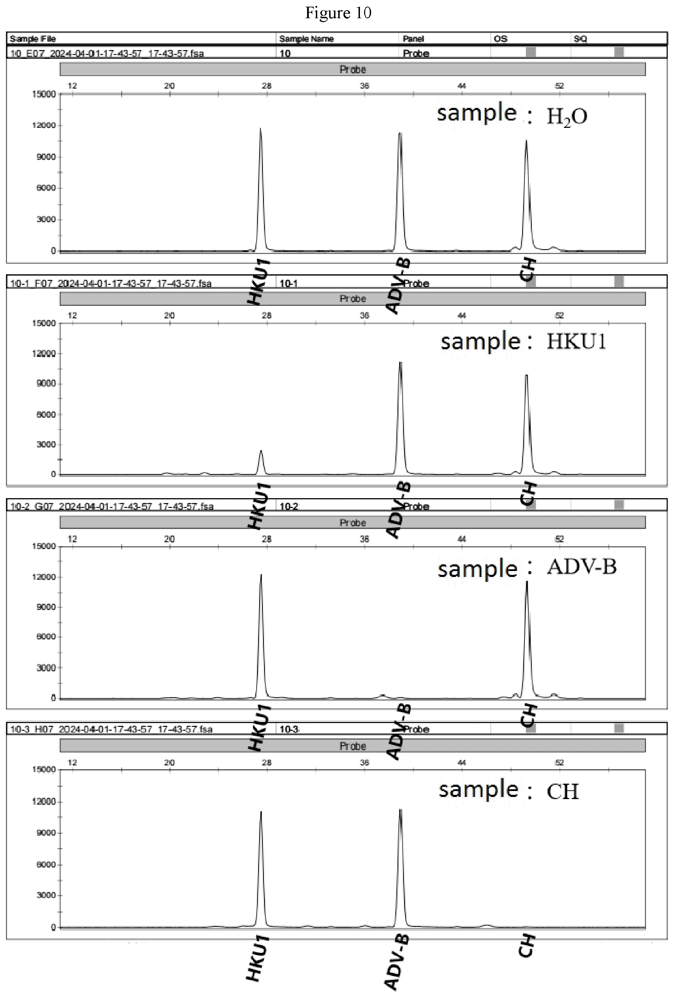

Figure 10 shows the detection effect of the present invention of the primer/probe composition for capillary electrophoresis of the present invention in Example 10, wherein 5' end of the nucleic acid probe carries a FAM fluorescent label, 3' end has a phosphorylation modification, and the length of the nucleic acid probe is 10 nt.

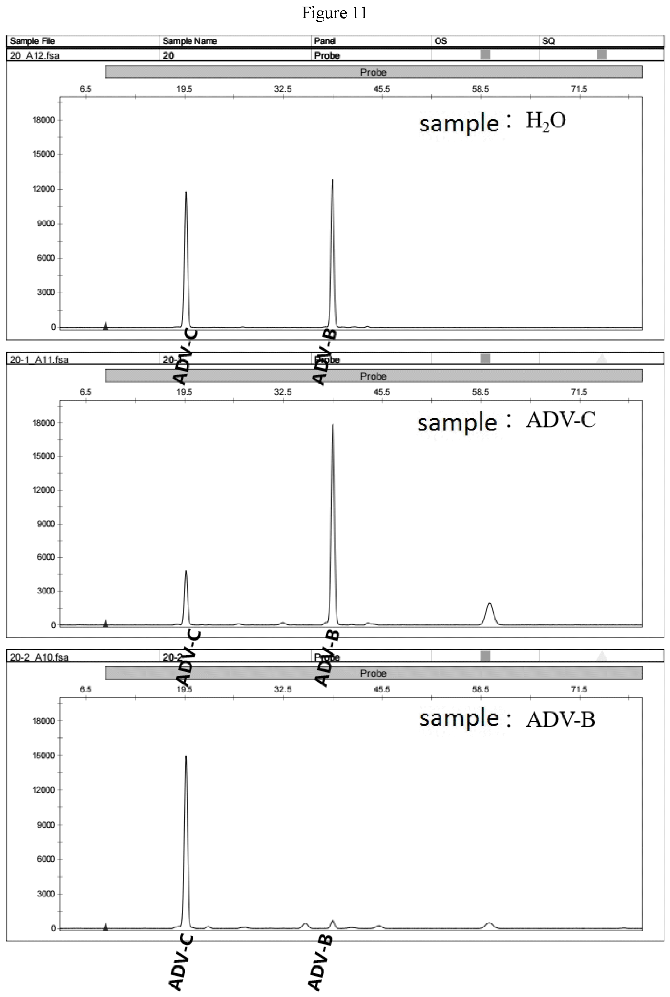

Figure 11 shows the detection effect of the present invention of the primer/probe composition for capillary electrophoresis of the present invention in Example 11, wherein 5' end of the nucleic acid probe carries a FAM fluorescent label, 3' end has a phosphorylation modification, and the length of the nucleic acid probe differs by 20 nt.

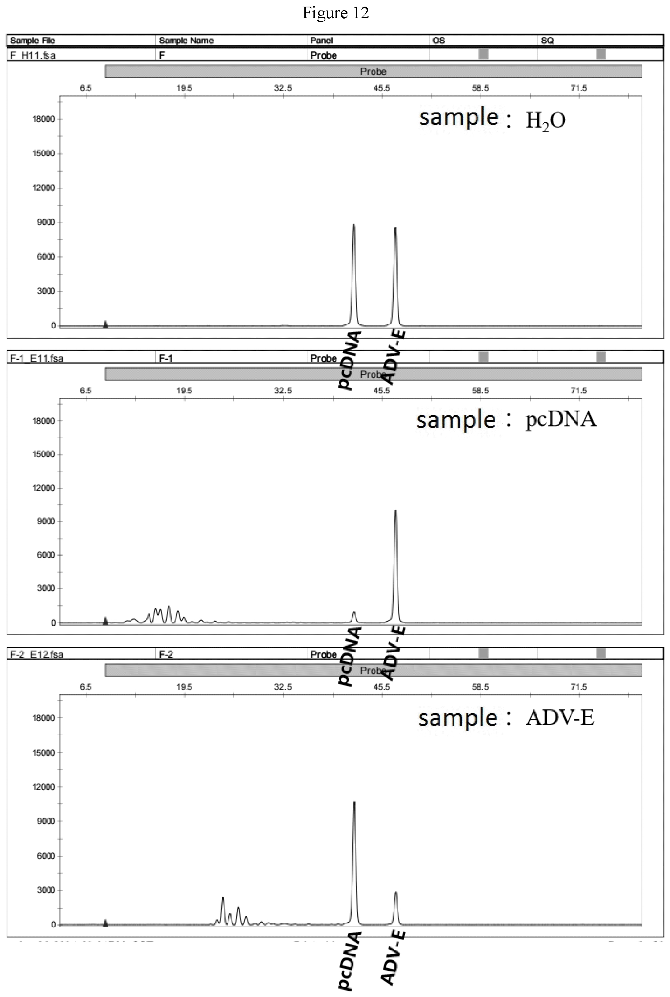

Figure 12 shows the detection effect of the present invention of the primer/probe composition for capillary electrophoresis of the present invention in Example 12, wherein 3' end of the nucleic acid probe carries a FAM fluorescent.

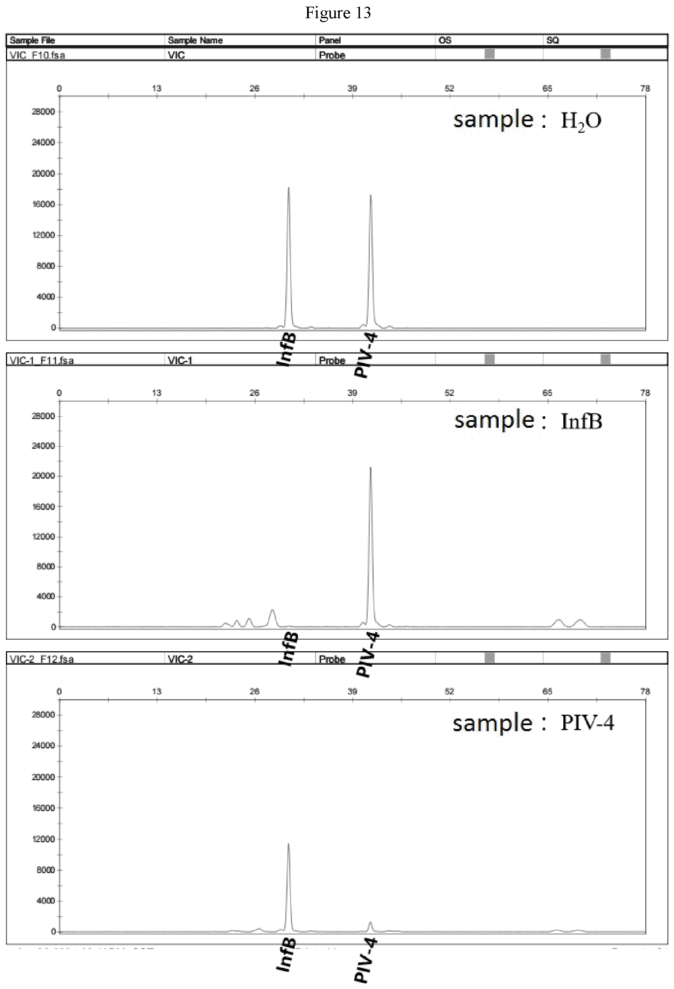

Figure 13 shows the detection effect of the present invention of the primer/probe composition for capillary electrophoresis of the present invention in Example 13, wherein 5' end of the nucleic acid probe carries a VIC fluorescent label, and 3' end has a phosphorylation modification.

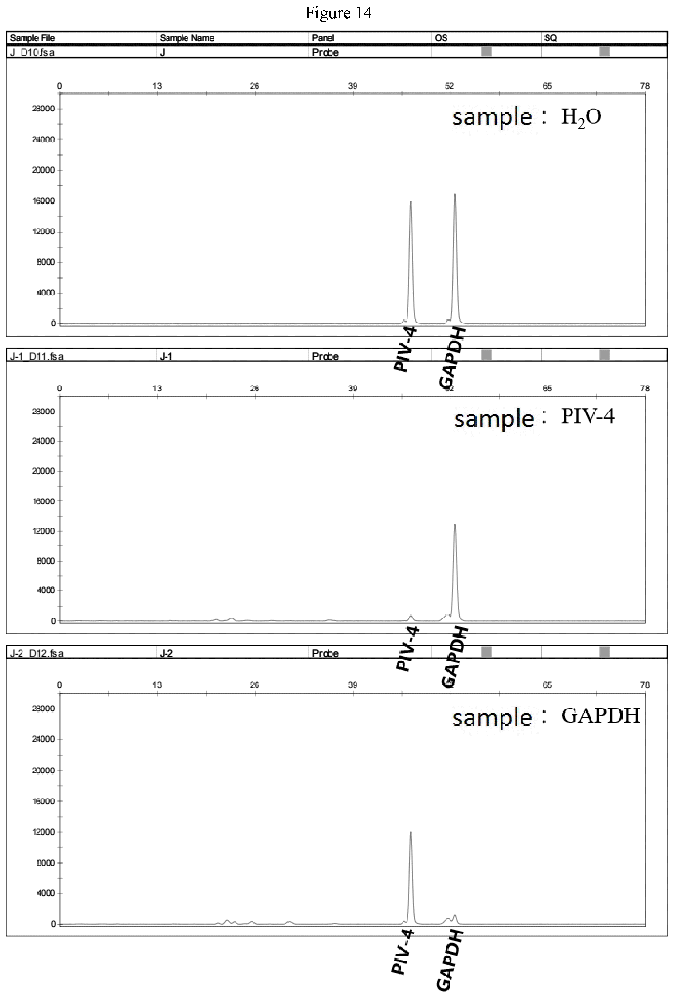

Figure 14 shows the detection effect of the present invention of the primer/probe composition for capillary electrophoresis of the present invention in Example 14, wherein 5' end of the nucleic acid probe carries a JOE fluorescent label.

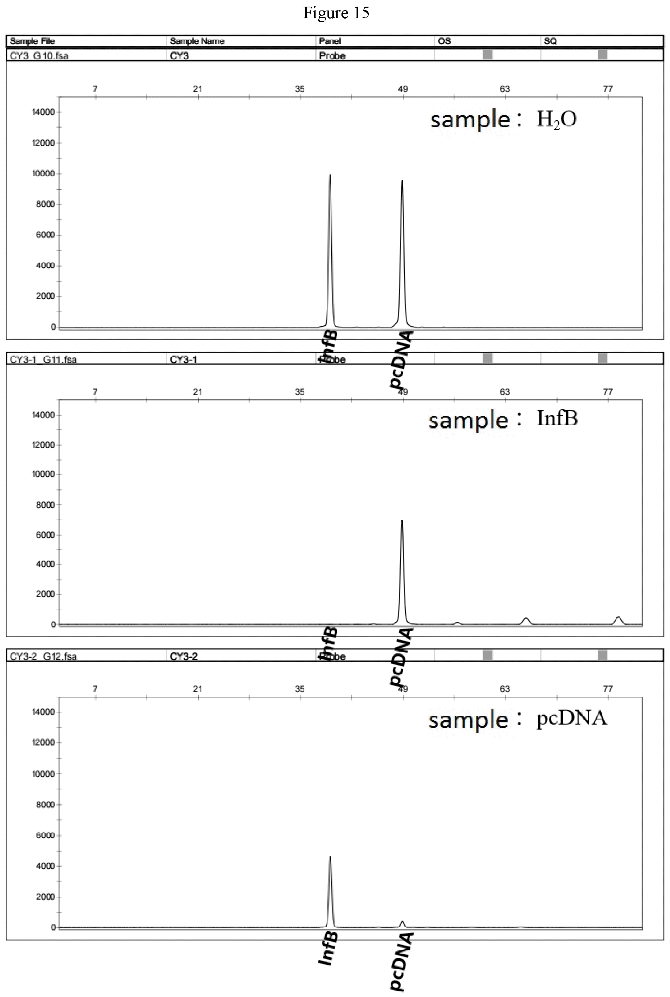

Figure 15 shows the detection effect of the present invention of the primer/probe composition for capillary electrophoresis of the present invention in Example 15, wherein 5' end of the nucleic acid probe carries a Cy3 fluorescent label, and 3' end has a phosphorylation modification.

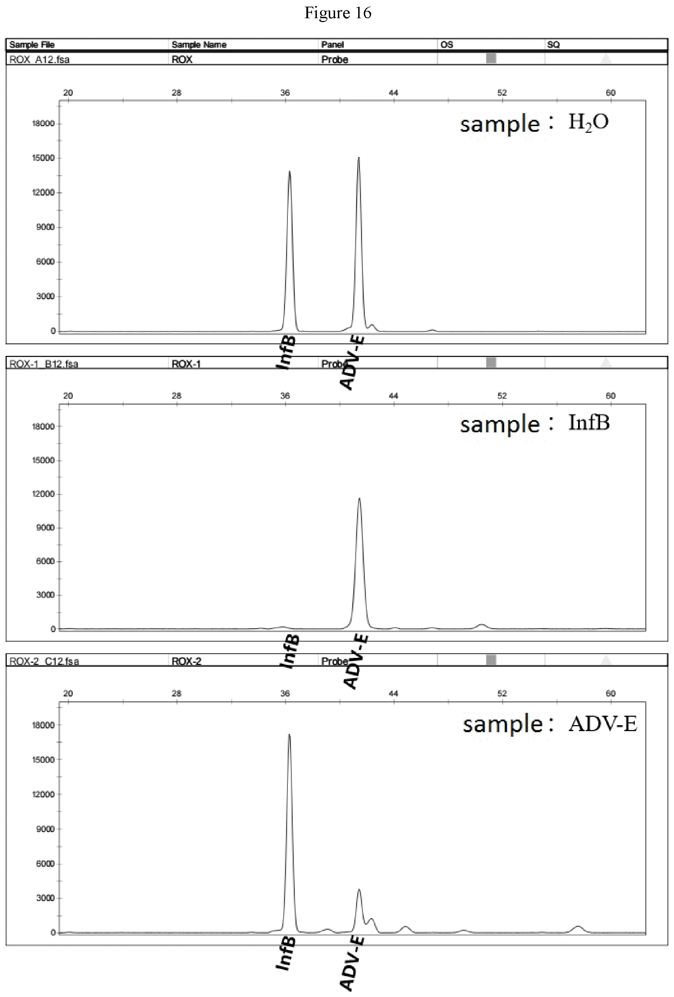

Figure 16 shows the detection effect of the present invention of the primer/probe composition for capillary electrophoresis of the present invention in Example 16, wherein 5' end of the nucleic acid probe carries a ROX fluorescent label, and 3' end has a phosphorylation modification.

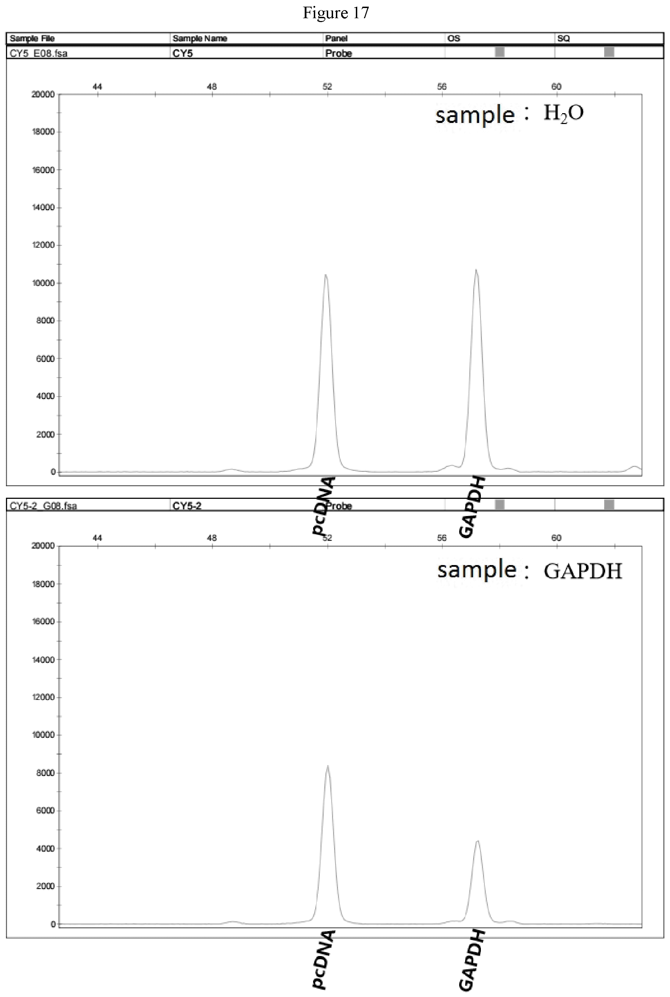

Figure 17 shows the detection effect of the present invention of the primer/probe composition for capillary electrophoresis of the present invention in Example 17, wherein 5' end of the nucleic acid probe carries a Cy5 fluorescent label, and and 3' end has a phosphorylation modification.

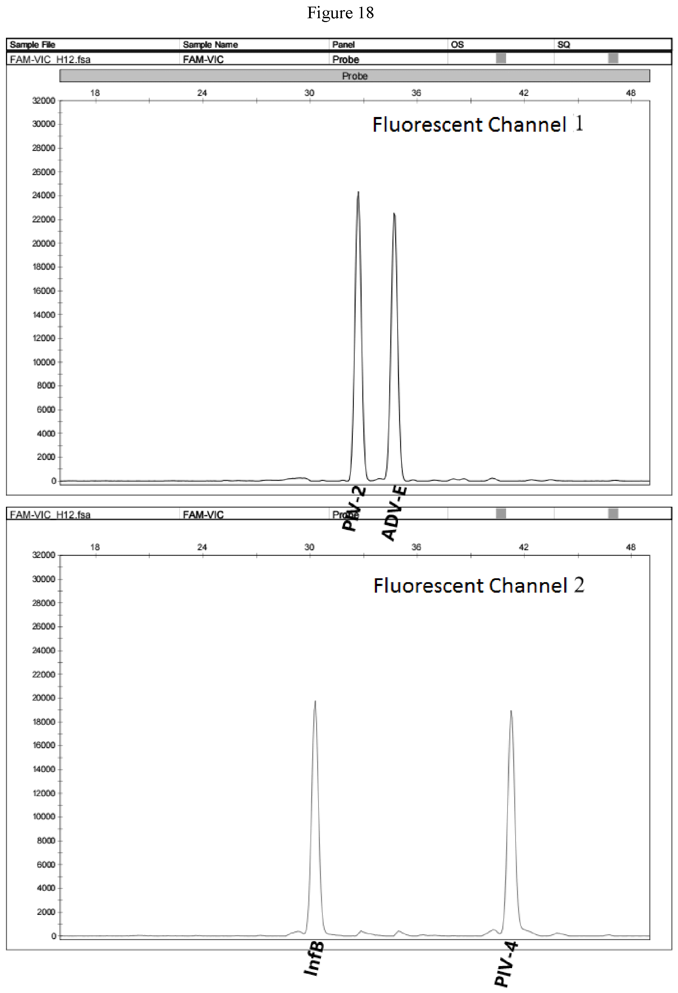

Figure 18 shows the effect of fluorescent signals of simultaneous electrophoresis of probes with different fluorescently labeled probes in Example 18.

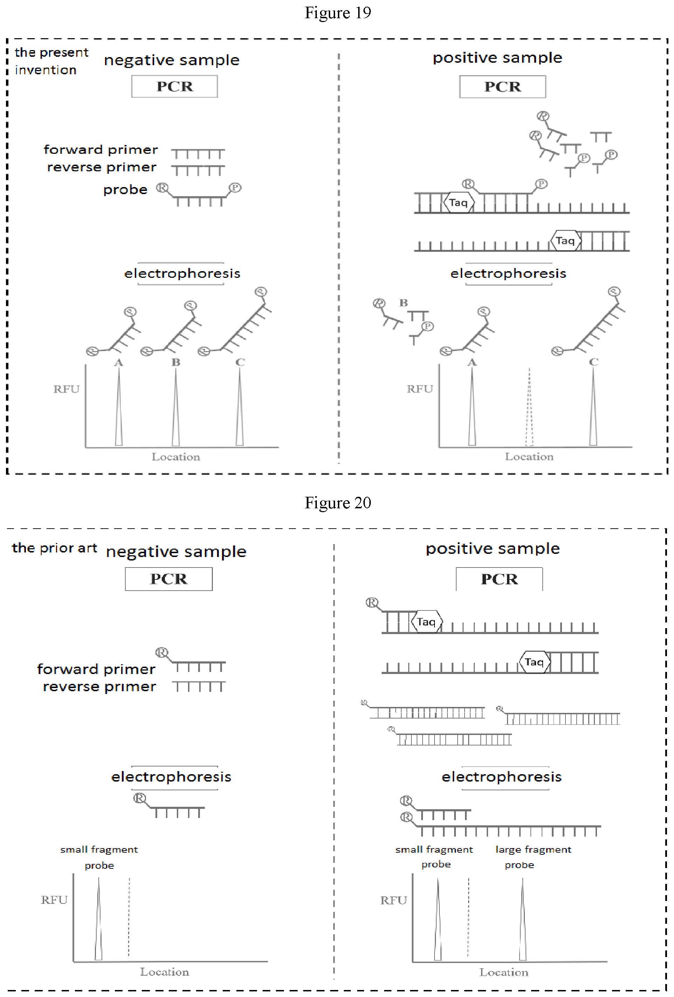

Figure 19 shows a schematic diagram of a primer/probe composition for capillary electrophoresis and a detection method according to the present invention.
Figure 20 shows a schematic diagram of a detection method in the prior art.

### BEST MODE FOR CARRYING OUT THE INVENTION

The present invention is further described below through specific examples, however, it should be understood that these examples are only used for more detailed description, and should not be construed to limit the present invention in any form.

This section provides a general description of the materials and test methods used in the tests of the present invention. While many of the materials and methods of operation used for the purposes of the present invention are known in the art, the present invention is described in as much detail as possible. It is clear to those skilled in the art that, in the context, if not specifically stated, the materials and methods of operation used in the present invention are well known in the art.

The following examples will be helpful for further understanding to the present invention, but these examples are not intended to limit the present invention.

The reagents and instruments used in the following examples are as follows:
Reagents:
   2*qRT-PCR Premix [v7] (with UDG) was purchased from Hangzhou Huadish Gold Compounding Biotechnology Co. Ltd. Hi-Di ^{™} Formamide was purchased from Thermo Scientific Technology Company; Nucleic Acid Extraction Kit (Magnetic Bead Method), Product ID: RT-A (SG) -200, purchased from Medium Cell Huji Biotechnology Co. Ltd.
Instrument:
   Electrophoresis Instrument, commercially available from Applied Biosystems, Model 3500 Dx.

In the primer/probe composition specifically used in Examples 1-18 below, the sequence of the forward primer thereof is as shown in SEQ ID NO. 1-16, the sequence of the reverse primer thereof is as shown in SEQ ID NO. 17-32, and the sequence of the nucleic acid probe is as shown in SEQ ID NO. 33-58:
SEQ ID NO. 1: GAAGCATGGTATGAATTTCGTG;
SEQ ID NO. 2: GGAGGAAGTAAACACTCAGAAAGA;
SEQ ID NO. 3: CTTGAACCATTGCAGTCACC;
SEQ ID NO. 4: TCTTCTAACCGAGGTCGAAACGTA;
SEQ ID NO. 5: CGTACGAACACTTTATCACCACTA;
SEQ ID NO. 6: CAGGCAAGTTAAGGTTAGATAGCA;
SEQ ID NO. 7: AGCCACAACACTTAAATTCACTC;
SEQ ID NO. 8: GACAGAACCCGGTATTTCAG;
SEQ ID NO. 9: CATATCTTAATCCGCAATACCACTC;
SEQ ID NO. 10: CTACTTCAGCAAATTTGACCCA;
SEQ ID NO. 11: CTCACCAGAAGTTGTTTGTATCAC;
SEQ ID NO. 12: TTAGGGTGGGAACTCTAACGA;
SEQ ID NO. 13: TCAGTAGTAGACCATGTGAATTCC;
SEQ ID NO. 14: TTCTCCGTTGGCTTAGTAAATGTC;
SEQ ID NO. 15: TTGATCTAGAAATTGCCCTCCT;
SEQ ID NO. 16: CTACAAGCGCGTGTATGATGAG;
SEQ ID NO. 17: ACCTGCACAATTACAGCCAA;
SEQ ID NO. 18: CCACTCTGGTCATATGCATTCAATCT;
SEQ ID NO. 19: CTCGTCTTGAAGGAAGTACAATCTA;
SEQ ID NO. 20: GTCTTGTCTTTAGCCATTCCA;
SEQ ID NO. 21: AGCCACTAATTGCATTACCAAG;
SEQ ID NO. 22: TGAAGATTAATGCGGCTTGTAG;
SEQ ID NO. 23: GATGAGCTAGTGTGCAAATGG;
SEQ ID NO. 24: AGTTTGGCAATTCATCCTCCAC;
SEQ ID NO. 25: CTAGCATCAGTTAACGGATTAATGG;
SEQ ID NO. 26: GCTTAATAGGCAATGCATTCCA;
SEQ ID NO. 27: GTTTAAGAGTCAGCGCGCAGTA;
SEQ ID NO. 28: CCATTACTAGCAATTCCGA;
SEQ ID NO. 29: CTGTGAATATGGGAGGTTTCATCA;
SEQ ID NO. 30: GGAGGTAAGCTCTAGTATGTAAGG;
SEQ ID NO. 31: CCAATTCGGTTCAGTCTAATCC;
SEQ ID NO. 32: CATGTCCTTATGCCGCTTTC;
SEQ ID NO. 33: FAM-ATGCATACAGCATCAACACCACACCACGACT-P (as the xml version of the sequence listing cannot reflect FAM-and P, the accurate specific sequence of SEQ ID NO. 33 is set forth in the description herein);
SEQ ID NO. 34: FAM-TAATGGAACATTCCTCAAACACCACCCCAATGG-P (as the xml version of the sequence listing cannot include FAM-and P, the accurate specific sequence of SEQ ID NO. 34 is set forth in the description herein);
SEQ ID NO. 35: FAM-ACAAAAATCCAGGAATCAGGGGTGATCCTCTAA-P (as the xml version of the sequence listing cannot include FAM-and P, the accurate specific sequence of SEQ ID NO. 35 is set forth in the description herein);
SEQ ID NO.36: FAM-TCAGGCCCCCTCAAAGCCGAGATCGCACAGAGAC-P (as the xml version of the sequence listing cannot include FAM-and P, the accurate specific sequence of SEQ ID NO. 36 is set forth in the description herein);
SEQ ID NO. 37: FAM-ATGTTCTTAAGCACGCTTAATCTCTATAGAGCATATGT-P (as the xml version of the sequence listing cannot include FAM-and P, the accurate specific sequence of SEQ ID NO. 37 is set forth in the description herein);
SEQ ID NO. 38: FAM-TTAGCAGACAAATTCCCAGTTCTTCAC-P (as the xml version of the sequence listing cannot include FAM-and P, the accurate specific sequence of SEQ ID NO. 38 is set forth in the description herein);
SEQ ID NO. 39: FAM-ATCATTTGAGACCTACCATACCCACCTATCAGGAC-P (as the xml version of the sequence listing cannot include FAM-and P, the accurate specific sequence of SEQ ID NO. 39 is set forth in the description herein);
SEQ ID NO. 40: FAM-TTCAATAATGCGCACATCAGGGATCATTAGCTGTCCACC-P (as the xml version of the sequence listing cannot include FAM-and P, the accurate specific sequence of SEQ ID NO. 40 is set forth in the description herein).
SEQ ID NO. 41: FAM-AAAAGCGCTGCATATGACCCAGTTA-P (as the xml version of the sequence listing cannot include FAM-and P, the accurate specific sequence of SEQ ID NO. 41 is set forth in the description herein).
SEQ ID NO. 42: FAM-TACATTGCATTTCATCAATTTGTTAGCACCTGACGATCAGTTAG-P (as the xml version of the sequence listing cannot include FAM-and P, the accurate specific sequence of SEQ ID NO. 42 is set forth in the description herein);
SEQ ID NO. 43: FAM-AAGACCATACTTCAGCGCACTCTCGAGGACGCCGAGCTCT-P (as the xml version of the sequence listing cannot include FAM-and P, the accurate specific sequence of SEQ ID NO. 43 is set forth in the description herein);
SEQ ID NO. 44: FAM-CTTTGAGATTTGCTCCAGTCTTACGATATTGCTACCTTCTGTACTG GCCA-P (as the xml version of the sequence listing cannot include FAM-and P, the accurate specific sequence of SEQ ID NO. 44 is set forth in the description herein);
SEQ ID NO: 45: FAM-AGAACTACAAATTATCACTTTGATACTAGCCCTATTAATCGCA-P (as the xml version of the sequence listing cannot include FAM-and P, the accurate specific sequence of SEQ ID NO. 45 is set forth in the description herein);
SEQ ID NO.46: FAM-CGGTCCTCGTCGCCGTACA-P (as the xml version of the sequence listing cannot include FAM-and P, the accurate specific sequence of SEQ ID NO. 46 is set forth in the description herein);
SEQ ID NO. 47: TCAGCTTAATGACAACTCGAGCCTTACTGGCGATGGTCCAA-FAM (as the xml version of the sequence listing cannot include FAM, the accurate specific sequence of SEQ ID NO. 47 is set forth in the description herein);
SEQ ID NO. 48: TTCAATAATGCGCACATCAGGGATCATTAGCTGTCCACC-FAM (as the xml version of the sequence listing cannot include FAM, the accurate specific sequence of SEQ ID NO. 48 is set forth in the description herein);
SEQ ID NO. 49: JOE-TACATTGCATTTCATCAATTTGTTAGCACCTGACGATCAGATCTT (as the xml version of the sequence listing cannot include JOE, the accurate specific sequence of SEQ ID NO. 49 is set forth in the description herein);
SEQ ID NO: 50: JOE-TAATAGTTATGTCATCCCTCTTATTAATCATCATCCTTAGCCCTTTC AGAC (as the xml version of the sequence listing cannot include JOE, the accurate specific sequence of SEQ ID NO. 50 is set forth in the description herein);
SEQ ID NO: 51: VIC-TACATTGCATTTCATCAATTTGTTAGCACCTGACGATCAGTTAG-P (as the xml version of the sequence listing cannot include VIC and P, the accurate specific sequence of SEQ ID NO. 51 is set forth in the description herein);
SEQ ID NO. 52: VIC-TAATGGAACATTCCTCAAACCCCAATGG-P (as the xml version of the sequence listing cannot include VIC and P, the accurate specific sequence of SEQ ID NO. 52 is set forth in the description herein);
SEQ ID NO: 53: Cy3-TCAGCTTAATGACAACTCGAGCCTTACTGGCGATGGTCCAA-P (as the xml version of the sequence listing cannot include Cy3 and P, the accurate specific sequence of SEQ ID NO. 53 is set forth in the description herein);
SEQ ID NO: 54: Cy3-TAATGGAACATTCCTCAAACACCACCCCAATGG-P (as the xml version of the sequence listing cannot include Cy3 and P, the accurate specific sequence of SEQ ID NO. 54 is set forth in the description herein);
SEQ ID NO. 55: ROX-TAATGGAACATTCCTCAAACCCCAATGG-P (as the xml version of the sequence listing cannot include ROX and P, the accurate specific sequence of SEQ ID NO. 55 is set forth in the description herein);
SEQ ID NO: 56: ROX-TTCAATAATGCGCACATCATGGATCATTAGCTGTCCACC-P (as the xml version of the sequence listing cannot include ROX and P, the accurate specific sequence of SEQ ID NO. 56 is set forth in the description herein);
SEQ ID NO: 57: CY5-TCAGCTTAATGACAACTCGAGCCTTACTGGCGATGGTCCAA-P (as the xml version of the sequence listing cannot include CY5 and P, the accurate specific sequence of SEQ ID NO. 57 is set forth in the description herein);
SEQ ID NO. 58: CY5-TAATAGTTATGTCATCCCTCTTATTAATCATCATCCTTAGCCCTTA AGTCT-P (as the xml version of the sequence listing cannot include CY5 and P, the accurate specific sequence of SEQ ID NO. 58 is set forth in the description herein).

In the following Examples 1-18, the standard substance SIZE is used to determine the electrophoretic mobility location, wherein the 5' end of which is the fluorescent labeled Alexa fluor 633, and the 3' end of which is a phosphorylation label. This standard substance for determining the electrophoretic migration position is composed of a plurality of nucleic acid sequences with fluorescent labels, and is mixed with the PCR amplification product in the electrophoresis pretreatment to achieve the effect of indicating the position of the nucleic acid probe in the electrophoresis process. The specific information is as shown in SEQ ID NO. 59-61:
SEQ ID NO: 59: Alexa fluor 633-ATAAGACTCGGCGGTTACTC-P (as the xml version of the sequence listing cannot include Alexa fluor 633 and P, the accurate specific sequence of SEQ ID NO. 59 is set forth in the description herein);
SEQ ID NO: 60: Alexa fluor 633-AGTGTAAGACTCGGCGGTTTGGTACTTCACACTACACCACG-P (as the xml version of the sequence listing cannot include Alexa fluor 633 and P, the accurate specific sequence of SEQ ID NO. 60 is set forth in the description herein);
SEQ ID NO. 61: Alexa fluor 633-TCCATCAGGACGGGGAATAACTATCTCCAACGTACATTGGCACTGATT TAGATGGTGACGACCTGTGCCG-P (as the xml version of the sequence listing cannot include Alexa fluor 633 and P, the accurate specific sequence of SEQ ID NO. 61 is set forth in the description herein).

### Example 1

This example is used to illustrate the primer/probe composition for capillary electrophoresis and the detection method of the present invention.

The sequence of the forward primer used in this example is SEQ ID NO.1, SEQ ID NO.2, SEQ ID NO: 3, the sequence of the reverse primer is SEQ ID NO: 17, SEQ ID NO: 18, SEQ ID NO: 19, and the sequence of the nucleic acid probe is SEQ ID NO: 33, SEQ ID NO: 34, SEQ ID NO: 35, and the fluorescent label used is FAM. The forward primers, reverse primers, and nucleic acid probes are synthesized by Shenggong bioengineering (Shanghai) Co. Ltd.
1. Sample: The to-be-tested sample HKU1 used in this example is a plasmid inserted by the coronavirus HKU1 fragment (purchased from Beijing Scientific Biotechnology Co. Ltd.); the sample InfB is an influenza B virus Victoria reference product (purchased from Guangzhou Steda Biotechnology Co. Ltd.); and the sample PIV-3 is a secondary influenza virus type 3 reference (purchased from Guangzhou Steda Biotechnology Co. Ltd.).
2. Pretreatment of a sample: The sample was subjected to nucleic acid extraction by using a nucleic acid extraction kit (magnetic bead method) (Product ID: RT-A (SG) -200, purchased from a medium-cell Huji Biotechnology Co. Ltd.), and the extraction step was performed according to the instruction of a nucleic acid extraction kit (magnetic bead method). The specific steps of the operation are as follows:
a) Preparation of working solution: mixing 500 µL of the extraction reagent I, 4 uL of the magnetic bead solution and 15 µL of the protease K;
b) Cracking: mixing the working solution with 200 µL of the sample, and cracking at 55°C for 4 min. The magnetic beads were adsorbed for 1 min, and the supernatant was discarded;
c) Adding 600 µL of extraction reagent II, and mixing well. The magnetic beads were adsorbed for 1 min, and the supernatant was discarded;
d) Dissolving the nucleic acid with 50 uL of eluent at 80° C. The magnetic beads were adsorbed for 30 s, and the magnetic beads were cleaned up for later use.

3. PCR Amplification:
The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 1. PCR reaction system**

| **Reagents** | **Amount (µL)** |
|---|---|
| 2*qRT-PCR Premix [v7] (with UDG) | 10 |
| Forward Primer (4µM) | 1 |
| Reverse Primer (4µM) | 1 |
| Nucleic Acid Probe (200nM) | 1 |
| Water | 2 |
| Template | 5 |
| Total Volume | 20 |

**TABLE 2. PCR reaction conditions**

| **Temperature** | **Time** | **Cycle Count** |
|---|---|---|
| 25°C | 2.5 min | 1 |
| 50°C | 10 min | 1 |
| 95°C | 2 min | 1 |
| 95°C | 10 s | 40 |
| 60°C | 90 s | |

4. Capillary electrophoresis
The electrophoresis premix was formulated according to the formulation of Table 3.

**TABLE 3. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of Table 4 using Applied Biosystems ^{™} 3500 Dx.

**TABLE 4. Preparation of Electrophoresis Solution**

| **Reagents** | **Amount** |
|---|---|
| Electrophoretic Premix | 9 µL |
| PCR amplified products | 1 µL |

**TABLE 5. Electrophoresis parameters**

| **Temperature (°C)** | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 1, the length of the nucleic acid probe differs by 1 nt, and the electrophoresis nucleic acid probe can be subjected to a zone division. When the detection sample is HKU1, the fluorescent signal peak of the nucleic acid probe corresponding to HKU1 is significantly reduced; when the detection sample is InfB, the fluorescent signal peak of the nucleic acid probe corresponding to InfB is significantly reduced; and when the detection sample is PIV -3, the fluorescent signal peak of the nucleic acid probe corresponding to PIV -3 is significantly reduced. In the prior art, whether the detection sample includes the detection target is determined by whether the fluorescent signal characteristic peak appears, and whether the detection sample includes the detection target is determined by whether the fluorescent signal characteristic peak is weakened.

### Example 2

This example is used to illustrate the primer/probe composition for capillary electrophoresis and the detection method of the present invention.

The sequence of the forward primer used in this example is SEQ ID NO: 1, SEQ ID NO: 3, SEQ ID NO: 4, and the sequence of the reverse primer is SEQ ID NO: 17, SEQ ID NO: 19, SEQ ID NO: 20, and the sequence of the nucleic acid probe is SEQ ID NO: 33, SEQ ID NO: 35, SEQ ID NO: 36, and the fluorescent label used is FAM. The forward primers, reverse primers, and nucleic acid probes are synthesized by Shenggong bioengineering (Shanghai) Co. Ltd.
1. Sample: The to-be-tested sample HKU1 used in this example is a plasmid inserted by the coronavirus HKU1 fragment (purchased from Beijing Scientific Biotechnology Co. Ltd.); the sample PIV -3 is a secondary influenza virus type 3 reference (purchased from Guangzhou Steda Biotechnology Co. Ltd.); and the sample InfA is an influenza A H1N1 reference (purchased from Guangzhou Steda Biotechnology Co. Ltd.).
2. Pretreatment of a sample: The sample was subjected to nucleic acid extraction by using a nucleic acid extraction kit (magnetic bead method) (Product ID: RT-A (SG) -200, purchased from a medium-cell Huji Biotechnology Co. Ltd.), and the extraction step was performed according to a nucleic acid extraction kit (magnetic bead method) specification. The specific steps of operation are the same as that in Example 1.
3. PCR Amplification:
The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 6. PCR reaction system**

| **Reagents** | **Amount (µL)** |
|---|---|
| 2*qRT-PCR Premix [v7] (with UDG) | 10 |
| Forward Primer (4µM) | 1 |
| Reverse Primer (4µM) | 1 |
| Nucleic Acid Probe (200nM) | 1 |
| Water | 2 |
| Template | 5 |
| Total Volume | 20 |

**TABLE 7. PCR reaction conditions**

| **Temperature** | **Time** | **Cycle Count** |
|---|---|---|
| 25°C | 2.5 min | 1 |
| 50°C | 10 min | 1 |
| 95°C | 2 min | 1 |
| 95°C | 10 s | 40 |
| 60°C | 90 s | |

4. Capillary electrophoresis
The electrophoresis premix was formulated according to the formulation of Table 8.

**TABLE 8. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of Table 9 using Applied Biosystems ^{™} 3500 Dx.

**TABLE 9. Preparation of Electrophoresis Solution**

| **Reagents** | **Amount** |
|---|---|
| Electrophoretic Premix | 9 µL |
| PCR amplified products | 1µL |

**TABLE 10. Electrophoresis parameters**

| **Temperature (°C)** | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 2, the nucleic acid probe has a difference of 2 nt from the length of the nucleic acid probe, and can be distinguished by an electrophoretic nucleic acid probe. When the detection sample is HKU1, the fluorescent signal peak of the nucleic acid probe corresponding to HKU1 is significantly reduced; when the detection sample is PIV -3, the fluorescent signal peak of the nucleic acid probe corresponding to PIV -3 is significantly reduced; and when the detection sample is InfA, the fluorescent signal peak of the nucleic acid probe corresponding to InfA is significantly reduced. Compared with the prior art, in the prior art, whether the detection sample includes the detection target is determined by whether the fluorescent signal characteristic peak appears, and whether the detection sample includes the detection target is determined by whether the fluorescent signal characteristic peak is weakened.

### Example 3

This example is used to illustrate the primer/probe composition for capillary electrophoresis and the detection method of the present invention.

The sequence of the forward primer used in this example is SEQ ID NO: 2, SEQ ID NO: 4, SEQ ID NO: 8, the sequence of the reverse primer is SEQ ID NO: 18, SEQ ID NO: 20, SEQ ID NO: 24, and the sequence of the nucleic acid probe is SEQ ID NO: 34, SEQ ID NO: 36, SEQ ID NO: 40, and the fluorescent label used is FAM. The forward primers, reverse primers, and nucleic acid probes are synthesized by Shenggong bioengineering (Shanghai) Co. Ltd.
1. Sample: The to-be-tested sample InfB used in this example is an influenza B virus Victoria reference product (purchased from Guangzhou Steda Biotechnology Co. Ltd.); the sample InfA is an influenza A virus H1N1 reference (purchased from Guangzhou Steda Biotechnology Co. Ltd.); and the sample ADV-E is an adenovirus type 4 reference (purchased from Guangzhou Steda Biotechnology Co. Ltd.).
2. Pretreatment of a sample: The sample was subjected to nucleic acid extraction by using a nucleic acid extraction kit (magnetic bead method) (Product ID: RT-A (SG) -200, purchased from a medium-cell Huji Biotechnology Co. Ltd.), and the extraction step was performed according to a nucleic acid extraction kit (magnetic bead method) specification. The specific steps of operation are the same as that in Example 1.
3. PCR Amplification:
The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 11. PCR reaction system**

| **Reagents** | **Amount (µL)** |
|---|---|
| 2*qRT-PCR Premix [v7] (with UDG) | 10 |
| Forward Primer (4µM) | 1 |
| Reverse Primer (4µM) | 1 |
| Nucleic Acid Probe (200nM) | 1 |
| Water | 2 |
| Template | 5 |
| Total Volume | 20 |

**TABLE 12: PCR reaction conditions:**

| **Temperature** | **Time** | **Cycle Count** |
|---|---|---|
| 25°C | 2.5 min | 1 |
| 50°C | 10 min | 1 |
| 95°C | 2 min | 1 |
| 95°C | 10 s | 40 |
| 60°C | 90 s | |

4. Capillary electrophoresis
The electrophoresis premix was formulated according to the formulation of Table 13.

**TABLE 13. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of Table 14 using Applied Biosystems ^{™} 3500 Dx.

**TABLE 14. Preparation of Electrophoresis Solution**

| **Reagents** | **Amount** |
|---|---|
| Electrophoretic Premix | 9 µL |
| PCR amplified products | 1µL |

**TABLE 15. Electrophoresis parameters**

| **Temperature (°C)** | **Injection Voltage(v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 3, the length of the nucleic acid probe is 3 nt, and the electrophoresis nucleic acid probe can be subjected to a zone division. When the detection sample is InfB, the fluorescent signal peak of the nucleic acid probe corresponding to InfB is significantly reduced; when the detection sample is InfA, the fluorescent signal peak of the nucleic acid probe corresponding to InfA is significantly reduced; and when the detection sample is ADV-E, the fluorescent signal peak of the nucleic acid probe corresponding to ADV-E is significantly reduced. Compared with the prior art, in the prior art, whether the detection sample includes the detection target is determined by whether the fluorescent signal characteristic peak appears, and whether the detection sample includes the detection target is determined by whether the fluorescent signal characteristic peak is weakened.

### Example 4

This example is used to illustrate the primer/probe composition for capillary electrophoresis and the detection method of the present invention.

The sequence of the forward primer used in this example is SEQ ID NO: 6, SEQ ID NO: 2, SEQ ID NO: 7, the sequence of the reverse primer is SEQ ID NO: 22, SEQ ID NO: 18, SEQ ID NO: 23, and the sequence of the nucleic acid probe is SEQ ID NO: 38, SEQ ID NO: 34, SEQ ID NO: 39, and the fluorescent label used is FAM. The forward primers, reverse primers, and nucleic acid probes are synthesized by Shenggong bioengineering (Shanghai) Co. Ltd.
1. Sample: The to-be-tested sample SARS-CoV -2 used in this example is a SARS-CoV -2 fragment inserted one.
2. The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 16. PCR reaction system**

| **Reagents** | **Amount (µL)** |
|---|---|
| 2*qRT-PCR Premix [v7] (with UDG) | 10 |
| Forward Primer (4µM) | 1 |
| Reverse Primer (4µM) | 1 |
| Nucleic Acid Probe (200nM) | 1 |
| Water | 2 |
| Template | 5 |
| Total Volume | 20 |

**TABLE 17: PCR reaction conditions:**

| **Temperature** | **Time** | **Cycle Count** |
|---|---|---|
| 25°C | 2.5 min | 1 |
| 50°C | 10 min | 1 |
| 95°C | 2 min | 1 |
| 95°C | 10 s | 40 |
| 60°C | 90 s | |

4. Capillary electrophoresis
The electrophoresis premix was formulated according to the formulation of Table 18.

**TABLE 18. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of Table 19 using Applied Biosystems ^{™} 3500 Dx.

**TABLE 19. Preparation of Electrophoresis Solution**

| **Reagents** | **Amount** |
|---|---|
| Electrophoretic Premix | 9 µL |
| PCR amplified products | 1µL |

**TABLE 20. Electrophoresis Parameters**

| **Temperature (°C)** | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 4, the length of the nucleic acid probe is 4 nt, and the electrophoresis nucleic acid probe can be distinguished. When the detection sample is SARS, the fluorescent signal peak of the nucleic acid probe corresponding to SARS is significantly reduced; when the detection sample is InfB, the fluorescent signal peak of the nucleic acid probe corresponding to InfB is significantly reduced; and when the detection sample is PIV -2, the fluorescent signal peak of the nucleic acid probe corresponding to PIV -2 is significantly reduced. Compared with the prior art, in the prior art, whether the detection sample includes the detection target is determined by whether the fluorescent signal characteristic peak appears, and whether the detection sample includes the detection target is determined by whether the fluorescent signal characteristic peak is weakened.

### Example 5

This example is used to illustrate the primer/probe composition for capillary electrophoresis and the detection method of the present invention.

The sequence of the forward primer used in this example is SEQ ID NO: 6, SEQ ID NO: 3, SEQ ID NO: 8, the sequence of the reverse primer is SEQ ID NO: 22, SEQ ID NO: 19, SEQ ID NO: 24, and the sequence of the nucleic acid probe is SEQ ID NO: 38, SEQ ID NO: 35, SEQ ID NO: 40, and the fluorescent label used is FAM. The forward primers, reverse primers, and nucleic acid probes are synthesized by Shenggong bioengineering (Shanghai) Co. Ltd.
1. Sample: The to-be-tested sample SARS-CoV -2 used in this example is a plasmid inserted by the SARS-CoV -2 fragment (purchased from Beijing Scientific Biotechnology Co. Ltd.); the sample PIV -3 is a secondary influenza virus type 3 reference product (purchased from Guangzhou Steda Biotechnology Co. Ltd.); and the sample ADV-E is an adenovirus type 4 reference (purchased from Guangzhou Steda Biotechnology Co. Ltd.).
2. Pretreatment of a sample: The sample was subjected to nucleic acid extraction by using a nucleic acid extraction kit (magnetic bead method) (Product ID: RT-A (SG) -200, purchased from a medium-cell Huji Biotechnology Co. Ltd.), and the extraction step was performed according to a nucleic acid extraction kit (magnetic bead method) specification. The specific steps of operation are the same as that in Example 1.
3. PCR Amplification:
The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 21. PCR reaction system**

| **Reagents** | **Amount (µL)** |
|---|---|
| 2*qRT-PCR Premix [v7] (with UDG) | 10 |
| Forward Primer (4µM) | 1 |
| Reverse Primer (4µM) | 1 |
| Nucleic Acid Probe (200nM) | 1 |
| Water | 2 |
| Template | 5 |
| Total Volume | 20 |

**TABLE 22. PCR reaction conditions:**

| **Temperature** | **Time** | **Cycle Count** |
|---|---|---|
| 25°C | 2.5 min | 1 |
| 50°C | 10 min | 1 |
| 95°C | 2 min | 1 |
| 95°C | 10 s | 40 |
| 60°C | 90 s | |

4. Capillary electrophoresis
The electrophoresis premix was formulated according to the formulation of Table 23.

**TABLE 23. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of Table 24 using Applied Biosystems ^{™} 3500 Dx.

**TABLE 24. Preparation of Electrophoresis Solution**

| **Reagents** | **Amount** |
|---|---|
| Electrophoretic Premix | 9 µL |
| PCR amplified products | 1 µL |

**TABLE 25. Electrophoresis parameters**

| **Temperature (°C)** | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 5, the length of the nucleic acid probe differs by 5 nt, and the electrophoresis nucleic acid probe can be subjected to regional separation. When the detection sample is SARS, the fluorescent signal peak of the nucleic acid probe corresponding to SARS is significantly reduced; when the detection sample is PIV -3, the fluorescent signal peak of the nucleic acid probe corresponding to PIV -3 is significantly reduced; and when the detection sample is ADV-E, the fluorescent signal peak of the nucleic acid probe corresponding to ADV-E is significantly reduced. Compared with the prior art, in the prior art, whether the detection sample includes the detection target is determined by whether the fluorescent signal characteristic peak appears, and whether the detection sample includes the detection target is determined by whether the fluorescent signal characteristic peak is weakened.

### Example 6

This example is used to illustrate the primer/probe composition for capillary electrophoresis and the detection method of the present invention.

The sequence of the forward primer used in this example is SEQ ID NO: 9, SEQ ID NO: 2, SEQ ID NO: 8, the sequence of the reverse primer is SEQ ID NO: 25, SEQ ID NO: 18, SEQ ID NO: 24, and the sequence of the nucleic acid probe is SEQ ID NO: 41, SEQ ID NO: 34, SEQ ID NO: 40, and the fluorescent label used is FAM. The forward primers, reverse primers, and nucleic acid probes are synthesized by Shenggong bioengineering (Shanghai) Co. Ltd.
1. Sample: The sample MP to be tested used in this example is a patient sample infected with a pneumonia branch; the sample InfB is an influenza B virus Victoria reference product (purchased from Guangzhou Steda Biotechnology Co. Ltd.); and the sample ADV-E is an adenovirus type 4 reference (purchased from Guangzhou Steda Biotechnology Co. Ltd.).
2. Pretreatment of a sample: The sample was subjected to nucleic acid extraction by using a nucleic acid extraction kit (magnetic bead method) (Product ID: RT-A (SG) -200, purchased from a medium-cell Huji Biotechnology Co. Ltd.), and the extraction step was performed according to a nucleic acid extraction kit (magnetic bead method) specification. The specific steps of operation are the same as that in Example 1.
3. PCR Amplification:
The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 26. PCR reaction system**

| **Reagents** | **Amount (µL)** |
|---|---|
| 2*qRT-PCR Premix [v7] (with UDG) | 10 |
| Forward Primer (4µM) | 1 |
| Reverse Primer (4µM) | 1 |
| Nucleic Acid Probe (200nM) | 1 |
| Water | 2 |
| Template | 5 |
| Total Volume | 20 |

**TABLE 27: PCR reaction conditions:**

| **Temperature** | **Time** | **Cycle Count** |
|---|---|---|
| 25°C | 2.5 min | 1 |
| 50°C | 10 min | 1 |
| 95°C | 2 min | 1 |
| 95°C | 10 s | 40 |
| 60 °C | 90 s | |

4. Capillary electrophoresis
The electrophoresis premix was formulated according to the formulation of Table 28.

**TABLE 28. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of Table 29 using Applied Biosystems ^{™} 3500 Dx.

**TABLE 29. Preparation of Electrophoresis Solution**

| **Reagents** | **Amount** |
|---|---|
| Electrophoretic Premix | 9 µL |
| PCR amplified products | 1µL |

**TABLE 30. Electrophoresis parameters**

| **Temperature (°C)** | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 6, the length of the nucleic acid probe is 6 nt, and the electrophoresis nucleic acid probe can be subjected to regional separation. When the detection sample is MP, the fluorescent signal peak of the nucleic acid probe corresponding to MP is significantly reduced; when the detection sample is InfB, the fluorescent signal peak of the nucleic acid probe corresponding to InfB is significantly reduced; when the detection sample is ADV-E, the fluorescent signal peak of the nucleic acid probe corresponding to ADV-E is significantly reduced. Compared with the prior art, in the prior art, whether the detection sample includes the detection target is determined by whether the fluorescent signal characteristic peak appears, and whether the detection sample includes the detection target is determined by whether the fluorescent signal characteristic peak is weakened.

### Example 7

This example is used to illustrate the primer/probe composition for capillary electrophoresis and the detection method of the present invention.

The sequence of the forward primer used in this example is SEQ ID NO: 6, SEQ ID NO: 4, SEQ ID NO: 10, the sequence of the reverse primer is SEQ ID NO: 22, SEQ ID NO: 20, SEQ ID NO: 26, and the sequence of the nucleic acid probe is SEQ ID NO: 38, SEQ ID NO: 36, SEQ ID NO: 42, and the fluorescent label used is FAM. The forward primers, reverse primers, and nucleic acid probes are synthesized by Shenggong bioengineering (Shanghai) Co. Ltd.
1. Sample: The to-be-tested sample SARS-CoV -2 used in this example is a plasmid inserted by the SARS-CoV -2 fragment (purchased from Beijing Scientific Biotechnology Co. Ltd.); the sample InfA is an influenza A virus H1N1 reference (purchased from Guangzhou Steda Biotechnology Co. Ltd.); and the sample PIV -4 is a secondary influenza virus type 4 reference (purchased from Guangzhou Steda Biotechnology Co. Ltd.).
2. Pretreatment of a sample: The sample was subjected to nucleic acid extraction by using a nucleic acid extraction kit (magnetic bead method) (Product ID: RT-A (SG) -200, purchased from a medium-cell Huji Biotechnology Co. Ltd.), and the extraction step was performed according to a nucleic acid extraction kit (magnetic bead method) specification. The specific steps of operation are the same as that in Example 1.
3. PCR Amplification:
The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 31. PCR reaction system**

| **Reagents** | **Amount (µL)** |
|---|---|
| 2*qRT-PCR Premix [v7] (with UDG) | 10 |
| Forward Primer (4µM) | 1 |
| Reverse Primer (4µM) | 1 |
| Nucleic Acid Probe (200nM) | 1 |
| Water | 2 |
| Template | 5 |
| Total Volume | 20 |

**TABLE 32: PCR reaction conditions**

| **Temperature** | **Time** | **Cycle Count** |
|---|---|---|
| 25°C | 2.5 min | 1 |
| 50°C | 10 min | 1 |
| 95°C | 2 min | 1 |
| 95°C | 10 s | 40 |
| 60°C | 90 s | |

4. Capillary electrophoresis
The electrophoresis premix was formulated according to the formulation of Table 33.

**TABLE 33. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of Table 34 using Applied Biosystems ^{™} 3500 Dx.

**TABLE 34. Preparation of Electrophoresis Solution**

| **Reagents** | **Amount** |
|---|---|
| Electrophoretic Premix | 9 µL |
| PCR amplified products | 1µL |

**TABLE 35. Electrophoresis Parameters**

| **Temperature (°C)** | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 7, the length of the nucleic acid probe is 7 nt, and the electrophoresis nucleic acid probe can be subjected to a zone division. When the detection sample is SARS, the fluorescent signal peak of the nucleic acid probe corresponding to SARS is significantly reduced; when the detection sample is InfA, the fluorescent signal peak of the nucleic acid probe corresponding to InfA is significantly reduced; and when the detection sample is PIV -4, the fluorescent signal peak of the nucleic acid probe corresponding to PIV -4 is significantly reduced. Compared with the prior art, in the prior art, whether the detection sample includes the detection target is determined by whether the fluorescent signal characteristic peak appears, and whether the detection sample includes the detection target is determined by whether the fluorescent signal characteristic peak is weakened.

### Example 8

This example is used to illustrate the primer/probe composition for capillary electrophoresis and the detection method of the present invention.

The sequence of the forward primer used in this example is SEQ ID NO: 6, SEQ ID NO: 7, SEQ ID NO: 13, the sequence of the reverse primer is SEQ ID NO: 22, SEQ ID NO: 23, SEQ ID NO: 29, and the sequence of the nucleic acid probe is SEQ ID NO: 38, SEQ ID NO: 39, SEQ ID NO: 45, and the fluorescent label used is FAM. The forward primers, reverse primers, and nucleic acid probes are synthesized by Shenggong bioengineering (Shanghai) Co. Ltd.
1. Sample: The to-be-tested sample SARS-CoV -2 used in this example is a plasmid inserted by the SARS-CoV -2 fragment (purchased from Beijing Scientific Biotechnology Co. Ltd.); the sample PIV -2 is a secondary influenza virus type 2 reference product (purchased from Guangzhou Steda Biotechnology Co. Ltd.); and the sample RSV-A is a respiratory syncytial virus A-type reference product (purchased from Guangzhou Steine Biotechnology Co. Ltd.).
2. Pretreatment of a sample: The sample was subjected to nucleic acid extraction by using a nucleic acid extraction kit (magnetic bead method) (Product ID: RT-A (SG) -200, purchased from a medium-cell Huji Biotechnology Co. Ltd.), and the extraction step was performed according to a nucleic acid extraction kit (magnetic bead method) specification. The specific steps of operation are the same as that in Example 1.
3. PCR Amplification:
The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 36. PCR reaction system**

| **Reagents** | **Amount (µL)** |
|---|---|
| 2*qRT-PCR Premix [v7] (with UDG) | 10 |
| Forward Primer (4µM) | 1 |
| Reverse Primer (4µM) | 1 |
| Nucleic Acid Probe (200nM) | 1 |
| Water | 2 |
| Template | 5 |
| Total Volume | 20 |

**TABLE 37: PCR reaction conditions**

| **Temperature** | **Time** | **Cycle Count** |
|---|---|---|
| 25°C | 2.5 min | 1 |
| 50°C | 10 min | 1 |
| 95°C | 2 min | 1 |
| 95°C | 10 s | 40 |
| 60°C | 90 s | |

4. Capillary electrophoresis
The electrophoresis premix was formulated according to the formulation of Table 38.

**TABLE 38. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of Table 39 using Applied Biosystems ^{™} 3500 Dx.

**TABLE 39. Preparation of Electrophoresis Solution**

| **Reagents** | **Amount** |
|---|---|
| Electrophoretic Premix | 9 µL |
| PCR amplified products | 1 µL |

**TABLE 40. Electrophoresis parameters**

| **Temperature (°C)** | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 8, the length of the nucleic acid probe is 8 nt, and the electrophoresis nucleic acid probe can be subjected to regional separation. When the detection sample is SARS, the fluorescent signal peak of the nucleic acid probe corresponding to SARS is significantly reduced; when the detection sample is PIV -2, the fluorescent signal peak of the nucleic acid probe corresponding to PIV -2 is significantly reduced; and when the detection sample is RSV-A, the fluorescent signal peak of the nucleic acid probe corresponding to RSV-A is significantly reduced. Compared with the prior art, in the prior art, whether the detection sample includes the detection target is determined by whether the fluorescent signal characteristic peak appears, and whether the detection sample includes the detection target is determined by whether the fluorescent signal characteristic peak is weakened.

### Example 9

This example is used to illustrate the primer/probe composition for capillary electrophoresis and the detection method of the present invention.

The sequence of the forward primer used in this example is SEQ ID NO: 9, SEQ ID NO: 4, SEQ ID NO: 13, the sequence of the reverse primer is SEQ ID NO: 25, SEQ ID NO: 20, SEQ ID NO: 29, and the sequence of the nucleic acid probe is SEQ ID NO: 41, SEQ ID NO: 36, SEQ ID NO: 45, and the fluorescent label used is FAM. The forward primers, reverse primers, and nucleic acid probes are synthesized by Shenggong bioengineering (Shanghai) Co. Ltd.
1. Sample: The sample MP to be tested used in this example is a patient sample infected with a pneumonia branch; the sample InfA is an influenza A virus H1N1 reference (purchased from Guangzhou Steda Biotechnology Co. Ltd.); and the sample RSV-A is a respiratory syncytial virus A-type reference (purchased from Guangzhou Steda Biotechnology Co. Ltd.).
2. Pretreatment of a sample: The sample was subjected to nucleic acid extraction by using a nucleic acid extraction kit (magnetic bead method) (Product ID: RT-A (SG) -200, purchased from a medium-cell Huji Biotechnology Co. Ltd.), and the extraction step was performed according to a nucleic acid extraction kit (magnetic bead method) specification. The specific steps of operation are the same as that in Example 1.
3. PCR Amplification:
The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 41. PCR reaction system**

| **Reagents** | **Amount (µL)** |
|---|---|
| 2*qRT-PCR Premix [v7] (with UDG) | 10 |
| Forward Primer (4µM) | 1 |
| Reverse Primer (4µM) | 1 |
| Nucleic Acid Probe (200nM) | 1 |
| Water | 2 |
| Template | 5 |
| Total Volume | 20 |

**TABLE 42: PCR reaction conditions**

| **Temperature** | **Time** | **Cycle Count** |
|---|---|---|
| 25°C | 2.5 min | 1 |
| 50°C | 10 min | 1 |
| 95°C | 2 min | 1 |
| 95°C | 10 s | 40 |
| 60°C | 90 s | |

4. Capillary electrophoresis
The electrophoresis premix was formulated according to the formulation of Table 43.

**TABLE 43. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of Table 44 using Applied Biosystems ^{™} 3500 Dx.

**TABLE 44. Preparation of Electrophoresis Solution**

| **Reagents** | **Amount** |
|---|---|
| Electrophoretic Premix | 9 µL |
| PCR amplified products | 1µL |

**TABLE 45. Electrophoresis Parameters**

| **Temperature (°C)** | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 9, the length of the nucleic acid probe is 9 nt, and the electrophoresis nucleic acid probe can be subjected to a zone division. When the detection sample is MP, the fluorescent signal peak of the nucleic acid probe corresponding to MP is significantly reduced; when the detection sample is InfA, the fluorescent signal peak of the nucleic acid probe corresponding to InfA is significantly reduced; when the detection sample is RSV-A, the fluorescent signal peak of the nucleic acid probe corresponding to RSV-A is significantly reduced. Compared with the prior art, in the prior art, whether the detection sample includes the detection target is determined by whether the fluorescent signal characteristic peak appears, and whether the detection sample includes the detection target is determined by whether the fluorescent signal characteristic peak is weakened.

### Example 10

This example is used to illustrate the primer/probe composition for capillary electrophoresis and the detection method of the present invention.

The sequence of the forward primer used in this example is SEQ ID NO: 1, SEQ ID NO: 11, SEQ ID NO: 12, and the sequence of the reverse primer is SEQ ID NO: 17, SEQ ID NO: 27, SEQ ID NO: 28, and the sequence of the nucleic acid probe is SEQ ID NO. 33, SEQ ID NO. 43, SEQ ID NO. 44, and the fluorescent label used is FAM. The forward primers, reverse primers, and nucleic acid probes are synthesized by Shenggong bioengineering (Shanghai) Co. Ltd.
1. Sample: The to-be-tested sample HKU1 used in this example is a plasmid inserted by the coronavirus HKU1 fragment (purchased from Beijing Scientific Biotechnology Co. Ltd.); the sample ADV-B is an adenovirus type 7 reference (purchased from Guangzhou Sham Biotechnology Co. Ltd.); and the sample CH is a plasmid inserted into the Chlamydia fragment (purchased from Beijing Scientific Biotechnology Co. Ltd.).
2. Pretreatment of a sample: The sample was subjected to nucleic acid extraction by using a nucleic acid extraction kit (magnetic bead method) (Product ID: RT-A (SG) -200, purchased from a medium-cell Huji Biotechnology Co. Ltd.), and the extraction step was performed according to a nucleic acid extraction kit (magnetic bead method) specification. The specific steps of operation are the same as that in Example 1.
3. PCR Amplification:
The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 46. PCR reaction system**

| **Reagents** | **Amount (µL)** |
|---|---|
| 2*qRT-PCR Premix [v7] (with UDG) | 10 |
| Forward Primer (4µM) | 1 |
| Reverse Primer (4µM) | 1 |
| Nucleic Acid Probe (200nM) | 1 |
| Water | 2 |
| Template | 5 |
| Total Volume | 20 |

**TABLE 47: PCR reaction conditions:**

| **Temperature** | **Time** | **Cycle Count** |
|---|---|---|
| 25°C | 2.5 min | 1 |
| 50°C | 10 min | 1 |
| 95°C | 2 min | 1 |
| 95°C | 10 s | 40 |
| 60°C | 90 s | |

4. Capillary electrophoresis
The electrophoresis premix was formulated according to the formulation of Table 48.

**TABLE 48. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of Table 49 using Applied Biosystems ^{™} 3500 Dx.

**TABLE 49: Electrophoresis Solution Formulation**

| **Reagents** | **Amount** |
|---|---|
| Electrophoretic Premix | 9 µL |
| PCR amplified products | 1µL |

**TABLE 50. Electrophoresis Parameters**

| **Temperature (°C)** | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 10, the length of the nucleic acid probe is 10 nt, and the electrophoresis nucleic acid probe can be distinguished. When the detection sample is HKU1, the fluorescent signal peak of the nucleic acid probe corresponding to HKU1 is significantly reduced; when the detection sample is ADV-B, the fluorescent signal peak of the nucleic acid probe corresponding to ADV-B is significantly reduced; when the detection sample is CH, the fluorescent signal peak of the nucleic acid probe corresponding to CH is significantly reduced. Compared with the prior art, in the prior art, whether the detection sample includes the detection target is determined by whether the fluorescent signal characteristic peak appears, and whether the detection sample includes the detection target is determined by whether the fluorescent signal characteristic peak is weakened.

### Example 11

This example is used to illustrate the primer/probe composition for capillary electrophoresis and the detection method of the present invention.

The sequence of the forward primer used in this example is SEQ ID NO: 16, SEQ ID NO: 11, the sequence of the reverse primer is SEQ ID NO: 32, SEQ ID NO: 27, the sequence of the nucleic acid probe is SEQ ID NO: 46, SEQ ID NO: 43, and the fluorescent label used is FAM. The forward primers, reverse primers, and nucleic acid probes are synthesized by Shenggong bioengineering (Shanghai) Co. Ltd.
1. Sample: The to-be-tested sample ADV-C used in this example is an adenovirus type 2 reference (purchased from Guangzhou Steda Biotechnology Co. Ltd.); and the sample ADV-B is an adenovirus type 7 reference (purchased from Guangzhou Steda Biotechnology Co. Ltd.).
2. Pretreatment of a sample: The sample was subjected to nucleic acid extraction by using a nucleic acid extraction kit (magnetic bead method) (Product ID: RT-A (SG) -200, purchased from a medium-cell Huji Biotechnology Co. Ltd.), and the extraction step was performed according to a nucleic acid extraction kit (magnetic bead method) specification. The specific steps of operation are the same as that in Example 1.
3. PCR Amplification:
The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 51. PCR reaction system**

| **Reagents** | **Amount (µL)** |
|---|---|
| 2*qRT-PCR Premix [v7] (with UDG) | 10 |
| Forward Primer (4µM) | 1 |
| Reverse Primer (4µM) | 1 |
| Nucleic Acid Probe (200nM) | 1 |
| Water | 2 |
| Template | 5 |
| Total Volume | 20 |

**TABLE 52. PCR reaction conditions:**

| **Temperature** | **Time** | **Cycle Count** |
|---|---|---|
| 25°C | 2.5 min | 1 |
| 50°C | 10 min | 1 |
| 95°C | 2 min | 1 |
| 95°C | 10 s | 40 |
| 60 °C | 90 s | |

4. Capillary electrophoresis
The electrophoresis premix was formulated according to the formulation of Table 53.

**TABLE 53. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of Table 54 using Applied Biosystems ^{™} 3500 Dx.

**TABLE 54. Preparation of Electrophoresis Solution**

| **Reagents** | **Amount** |
|---|---|
| Electrophoretic Premix | 9 µL |
| PCR amplified products | 1µL |

**TABLE 55. Electrophoresis Parameters**

| **Temperature (°C)** | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 11, the length of the nucleic acid probe is 20 nt, and the electrophoresis nucleic acid probe can be subjected to a zone division. When the detection sample is ADV-C, the fluorescent signal peak of the nucleic acid probe corresponding to ADV-C is significantly reduced; when the detection sample is ADV-B, the fluorescent signal peak of the nucleic acid probe corresponding to ADV-B is significantly reduced. Compared with the prior art, in the prior art, whether the detection sample includes the detection target is determined by whether the fluorescent signal characteristic peak appears, and whether the detection sample includes the detection target is determined by whether the fluorescent signal characteristic peak is weakened.

### Example 12

This example is used to illustrate the primer/probe composition for capillary electrophoresis and the detection method of the present invention.

The sequence of the forward primer used in this example is SEQ ID NO: 14, SEQ ID NO: 8, the sequence of the reverse primer is SEQ ID NO: 30, SEQ ID NO: 24, the sequence of the nucleic acid probe is SEQ ID NO: 47, SEQ ID NO: 48, and the fluorescent label used is FAM. The forward primers, reverse primers, and nucleic acid probes are synthesized by Shenggong bioengineering (Shanghai) Co. Ltd.
1. Sample: The sample pcDNA to be tested used in this example is a plasmid inserted by a random fragment (purchased from Beijing Scientific Biotechnology Co. Ltd.); and the sample ADV-E is an adenovirus type 4 reference (purchased from Guangzhou Steda Biotechnology Co. Ltd.).
2. Pretreatment of a sample: The sample was subjected to nucleic acid extraction by using a nucleic acid extraction kit (magnetic bead method) (Product ID: RT-A (SG) -200, purchased from a medium-cell Huji Biotechnology Co. Ltd.), and the extraction step was performed according to a nucleic acid extraction kit (magnetic bead method) specification. The specific steps of operation are the same as that in Example 1.
3. PCR Amplification:
The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 56. PCR reaction system**

| **Reagents** | **Amount (µL)** |
|---|---|
| 2*qRT-PCR Premix [v7] (with UDG) | 10 |
| Forward Primer (4µM) | 1 |
| Reverse Primer (4µM) | 1 |
| Nucleic Acid Probe (200nM) | 1 |
| Water | 2 |
| Template | 5 |
| Total Volume | 20 |

**TABLE 57: PCR reaction conditions:**

| **Temperature** | **Time** | **Cycle Count** |
|---|---|---|
| 25°C | 2.5 min | 1 |
| 50°C | 10 min | 1 |
| 95°C | 2 min | 1 |
| 95°C | 10 s | 40 |
| 60°C | 90 s | |

4. Capillary electrophoresis
The electrophoresis premix was formulated according to the formulation of Table 58.

**TABLE 58. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of Table 59 using Applied Biosystems ^{™} 3500 Dx.

**TABLE 59. Preparation of Electrophoresis Solution**

| **Reagents** | **Amount** |
|---|---|
| Electrophoretic Premix | 9 µL |
| PCR amplified products | 1 µL |

**TABLE 60. Electrophoresis Parameters**

| **Temperature (°C)** | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 12, the nucleic acid probe fluorescent label may be distinguished by electrophoresis nucleic acid probes at 3 ' . When the detection sample is pcDNA, the fluorescent signal peak of the nucleic acid probe corresponding to pcDNA is significantly reduced; when the detection sample is ADV-E, the fluorescent signal peak of the nucleic acid probe corresponding to ADV-E is significantly reduced. In the prior art, whether the detection sample includes the detection target is determined by whether the fluorescent signal characteristic peak appears, and whether the detection sample includes the detection target is determined by whether the fluorescent signal characteristic peak is weakened.

### Example 13

This example is used to illustrate the primer/probe composition for capillary electrophoresis and the detection method of the present invention.

The sequence of the forward primer used in this example is SEQ ID NO: 2, SEQ ID NO: 10, the sequence of the reverse primer is SEQ ID NO: 18, SEQ ID NO: 26, the sequence of the nucleic acid probe is SEQ ID NO: 51, SEQ ID NO: 52, and the fluorescent label used is VIC. The forward primers, reverse primers, and nucleic acid probes are synthesized by Shenggong bioengineering (Shanghai) Co. Ltd.
1. Sample: The to-be-tested sample InfB used in this example is an influenza B virus Victoria series reference (purchased from Guangzhou Steda Biotechnology Co. Ltd.); and the sample PIV -4 is a secondary influenza virus type 4 reference (purchased from Guangzhou Steda Biotechnology Co. Ltd.).
2. Pretreatment of a sample: The sample was subjected to nucleic acid extraction by using a nucleic acid extraction kit (magnetic bead method) (Product ID: RT-A (SG) -200, purchased from a medium-cell Huji Biotechnology Co. Ltd.), and the extraction step was performed according to a nucleic acid extraction kit (magnetic bead method) specification. The specific steps of operation are the same as that in Example 1.
3. PCR Amplification:
The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 61. PCR reaction system**

| **Reagents** | **Amount (µL)** |
|---|---|
| 2*qRT-PCR Premix [v7] (with UDG) | 10 |
| Forward Primer (4µM) | 1 |
| Reverse Primer (4µM) | 1 |
| Nucleic Acid Probe (200nM) | 1 |
| Water | 2 |
| Template | 5 |
| Total Volume | 20 |

**TABLE 62. PCR reaction conditions:**

| **Temperature** | **Time** | **Cycle Count** |
|---|---|---|
| 25°C | 2.5 min | 1 |
| 50°C | 10 min | 1 |
| 95°C | 2 min | 1 |
| 95°C | 10 s | 40 |
| 60°C | 90 s | |

4. Capillary electrophoresis
The electrophoresis premix was formulated according to the formulation of Table 63.

**TABLE 63. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of Table 64 using Applied Biosystems ^{™} 3500 Dx.

**TABLE 64. Preparation of Electrophoresis Solution**

| **Reagents** | **Amount** |
|---|---|
| Electrophoretic Premix | 9 µL |
| PCR amplified products | 1µL |

**TABLE 65. Electrophoresis parameters**

| **Temperature (°C)** | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 13, when the nucleic acid probe is VIC, an electrophoretic nucleic acid probe may be subjected to a zone division. When the detection sample is InfB, the fluorescent signal peak of the nucleic acid probe corresponding to InfB is significantly reduced; and when the detection sample is PIV -4, the fluorescent signal peak of the nucleic acid probe corresponding to PIV -4 is significantly reduced. Compared with the prior art, in the prior art, whether the detection sample includes the detection target is determined by whether the fluorescent signal characteristic peak appears, and whether the detection sample includes the detection target is determined by whether the fluorescent signal characteristic peak is weakened.

### Example 14

This example is used to illustrate the primer/probe composition for capillary electrophoresis and the detection method of the present invention.

The sequence of the forward primer used in this example is SEQ ID NO: 10, SEQ ID NO: 15, the sequence of the reverse primer is SEQ ID NO: 26, SEQ ID NO: 31, the sequence of the nucleic acid probe is SEQ ID NO: 49, SEQ ID NO: 50, and the fluorescent label used is JOE. The forward primers, reverse primers, and nucleic acid probes are synthesized by Shenggong bioengineering (Shanghai) Co. Ltd.
1. Sample: The to-be-tested sample PIV -4 used in this example is a secondary influenza virus type 4 reference (purchased from Guangzhou Steda Biotechnology Co. Ltd.); and the sample GAPDH is a pharyngeal swab sample.
2. Pretreatment of a sample: The sample was subjected to nucleic acid extraction by using a nucleic acid extraction kit (magnetic bead method) (Product ID: RT-A (SG) -200, purchased from a medium-cell Huji Biotechnology Co. Ltd.), and the extraction step was performed according to a nucleic acid extraction kit (magnetic bead method) specification. The specific steps of operation are the same as that in Example 1.
3. PCR Amplification:
The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 66. PCR reaction system**

| **Reagents** | **Amount (µL)** |
|---|---|
| 2*qRT-PCR Premix [v7] (with UDG) | 10 |
| Forward Primer (4µM) | 1 |
| Reverse Primer (4µM) | 1 |
| Nucleic Acid Probe (200nM) | 1 |
| Water | 2 |
| Template | 5 |
| Total Volume | 20 |

**TABLE 67. PCR reaction conditions:**

| **Temperature** | **Time** | **Cycle Count** |
|---|---|---|
| 25°C | 2.5 min | 1 |
| 50°C | 10 min | 1 |
| 95°C | 2 min | 1 |
| 95°C | 10 s | 40 |
| 60°C | 90 s | |

4. Capillary electrophoresis
The electrophoresis premix was formulated according to the formulation of Table 68.

**TABLE 68. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of Table 69 using Applied Biosystems ^{™} 3500 Dx.

**TABLE 69. Preparation of Electrophoresis Solution**

| **Reagents** | **Amount** |
|---|---|
| Electrophoretic Premix | 9 µL |
| PCR amplified products | 1µL |

**TABLE 70. Electrophoresis Parameters**

| **Temperature** (°C) | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 14, the fluorescent label of the nucleic acid probe 5 ' is JOE, and 3 ' is a non-complementary nucleotide that passes through the electrophoretic nucleic acid probe. When the detection sample is PIV -4, the fluorescent signal peak of the nucleic acid probe corresponding to PIV -4 is significantly reduced; and when the detection sample is GAPDH, the fluorescent signal peak of the nucleic acid probe corresponding to GAPDH is significantly reduced. Compared with the prior art, in the prior art, whether the detection sample includes the detection target is determined by whether the fluorescent signal characteristic peak appears, and whether the detection sample includes the detection target is determined by whether the fluorescent signal characteristic peak is weakened.

### Example 15

This example is used to illustrate the primer/probe composition for capillary electrophoresis and the detection method of the present invention.

The sequence of the forward primer used in this example is SEQ ID NO: 2, SEQ ID NO: 14, the sequence of the reverse primer is SEQ ID NO: 18, SEQ ID NO: 30, the sequence of the nucleic acid probe is SEQ ID NO: 53, SEQ ID NO: 54, the fluorescent label used is the Cy3. The forward primers, reverse primers, and nucleic acid probes are synthesized by Shenggong bioengineering (Shanghai) Co. Ltd.
1. Sample sample: The sample InfB to be tested used in this example is an influenza B virus Victoria reference product (purchased from Guangzhou Steda Biotechnology Co. Ltd.); and the sample pcDNA is a plasmid inserted into a random fragment (purchased from Beijing Scientific Biotechnology Co. Ltd.).
2. Pretreatment of a sample: The sample was subjected to nucleic acid extraction by using a nucleic acid extraction kit (magnetic bead method) (Product ID: RT-A (SG) -200, purchased from a medium-cell Huji Biotechnology Co. Ltd.), and the extraction step was performed according to a nucleic acid extraction kit (magnetic bead method) specification. The specific steps of operation are the same as that in Example 1.
3. PCR Amplification:
The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 71. PCR reaction system**

| **Reagents** | **Amount (µL)** |
|---|---|
| 2*qRT-PCR Premix [v7] (with UDG) | 10 |
| Forward Primer (40µM) | 1 |
| Reverse Primer (40µM) | 1 |
| Nucleic Acid Probe (2.4µM) | 1 |
| Water | 2 |
| Template | 5 |
| Total Volume | 20 |

**TABLE 72. PCR reaction conditions:**

| **Temperature** | **Time** | **Cycle Count** |
|---|---|---|
| 25°C | 2.5 min | 1 |
| 50°C | 10 min | 1 |
| 95 °C | 2 min | 1 |
| 95°C | 10 s | 40 |
| 60 °C | 90 s | |

4. Capillary electrophoresis
The electrophoresis premix was formulated according to the formulation of Table 73.

**TABLE 73. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of table 74 using Applied Biosystems ^{™} 3500 Dx.

**TABLE 74. Preparation of Electrophoresis Solution**

| **Reagents** | **Amount** |
|---|---|
| Electrophoretic Premix | 9 µL |
| PCR amplified products | 1 µL |

**TABLE 75. Electrophoresis Parameters**

| **Temperature** (°C) | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 15, when the nucleic acid probe fluorescent label is Cy3, the electrophoretic nucleic acid probe can be subjected to a region division. When the detection sample is InfB, the fluorescent signal peak of the nucleic acid probe corresponding to InfB is significantly reduced; when the detection sample is pcDNA, the fluorescent signal peak of the nucleic acid probe corresponding to pcDNA is significantly reduced. Compared with the prior art, in the prior art, whether the detection sample includes the detection target is determined by whether the fluorescent signal characteristic peak appears, and whether the detection sample includes the detection target is determined by whether the fluorescent signal characteristic peak is weakened.

### Example 16

This example is used to illustrate the primer/probe composition for capillary electrophoresis and the detection method of the present invention.

The sequence of the forward primer used in this example is SEQ ID NO: 2, SEQ ID NO: 8, the sequence of the reverse primer is SEQ ID NO: 18, SEQ ID NO: 24, the sequence of the nucleic acid probe is SEQ ID NO: 55, SEQ ID NO: 56, and the fluorescent label used is ROX. The forward primers, reverse primers, and nucleic acid probes are synthesized by Shenggong bioengineering (Shanghai) Co. Ltd.
1. Sample: The to-be-tested sample InfB used in this example is an influenza B virus Victoria series reference (purchased from Guangzhou Steda Biotechnology Co. Ltd.); and the sample ADV-E is an adenovirus type 4 reference (purchased from Guangzhou Steda Biotechnology Co. Ltd.).
2. Pretreatment of a sample: The sample was subjected to nucleic acid extraction by using a nucleic acid extraction kit (magnetic bead method) (Product ID: RT-A (SG) -200, purchased from a medium-cell Huji Biotechnology Co. Ltd.), and the extraction step was performed according to a nucleic acid extraction kit (magnetic bead method) specification. The specific steps of operation are the same as that in Example 1.
3. PCR Amplification:
The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 76. PCR reaction system**

| **Reagents** | **Amount (µL)** |
|---|---|
| 2*qRT-PCR Premix [v7] (with UDG) | 10 |
| Forward Primer (30µM) | 1 |
| Reverse Primer (30µM) | 1 |
| Nucleic Acid Probe (2µM) | 1 |
| Water | 2 |
| Template | 5 |
| Total Volume | 20 |

**TABLE 77: PCR reaction conditions:**

| **Temperature** | **Time** | **Cycle Count** |
|---|---|---|
| 25°C | 2.5 min | 1 |
| 50°C | 10 min | 1 |
| 95 °C | 2 min | 1 |
| 95°C | 10 s | 40 |
| 60°C | 90 s | |

4. Capillary electrophoresis
The electrophoresis premix was formulated according to the formulation of Table 78.

**TABLE 78. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of Table 79 using Applied Biosystems ^{™} 3500 Dx.

**TABLE 79. Preparation of Electrophoresis Solution**

| **Reagents** | **Amount** |
|---|---|
| Electrophoretic Premix | 9 µL |
| PCR amplified products | 1µL |

**TABLE 80. Electrophoresis parameters**

| **Temperature (°C)** | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 16, when the nucleic acid probe fluorescent label is ROX, an electrophoretic nucleic acid probe can be subjected to a region. When the detection sample is InfB, the fluorescent signal peak of the nucleic acid probe corresponding to InfB is significantly reduced; when the detection sample is ADV-E, the fluorescent signal peak of the nucleic acid probe corresponding to ADV-E is significantly reduced. Compared with the prior art, in the prior art, whether the detection sample includes the detection target is determined by whether the fluorescent signal characteristic peak appears, and whether the detection sample includes the detection target is determined by whether the fluorescent signal characteristic peak is weakened.

### Example 17

This example is used to illustrate the primer/probe composition for capillary electrophoresis and the detection method of the present invention.

The sequence of the forward primer used in this example is SEQ ID NO: 15, the sequence of the reverse primer is SEQ ID NO: 31, the sequence of the nucleic acid probe is SEQ ID NO. 57, SEQ ID NO. 58, the fluorescent label used is the Cy5. The forward primers, reverse primers, and nucleic acid probes are synthesized by Shenggong bioengineering (Shanghai) Co. Ltd.
1. Sample: Sample GAPDH is a pharyngeal swab sample (provided by the Ningbo Specialized People Hospital).
2. Pretreatment of a sample: The sample was subjected to nucleic acid extraction by using a nucleic acid extraction kit (magnetic bead method) (Product ID: RT-A (SG) -200, purchased from a medium-cell Huji Biotechnology Co. Ltd.), and the extraction step was performed according to a nucleic acid extraction kit (magnetic bead method) specification. The specific steps of operation are the same as that in Example 1.
3. PCR Amplification:
The PCR reaction system was configured: the buffer (2 * qRT-PCR Premix [v7] (with UDG)) was mixed and the forward primer, reverse primer, nucleic acid probe and template were added according to the PCR reaction system. After mixing, PCR amplification was performed in a PCR amplification instrument.

**TABLE 81. PCR reaction system**

| **Reagents** | **Amount (µL)** |
|---|---|
| 2*qRT-PCR Premix [v7] (with UDG) | 10 |
| Forward Primer (60µM) | 1 |
| Reverse Primer (60µM) | 1 |
| Nucleic Acid Probe (5µM) | 1 |
| Water | 2 |
| Template | 5 |
| Total Volume | 20 |

**TABLE 82: PCR reaction conditions:**

| **Temperature** | **Time** | **Cycle Count** |
|---|---|---|
| 25°C | 2.5 min | 1 |
| 50°C | 10 min | 1 |
| 95 °C | 2 min | 1 |
| 95°C | 10 s | 40 |
| 60 °C | 90 s | |

4. Capillary electrophoresis
The electrophoresis premix was formulated according to the formulation in Table 83.

**TABLE 83. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR amplified products were formulated according to the formulation of Table 84 using Applied Biosystems ^{™} 3500 Dx.

**TABLE 84. Preparation of Electrophoresis Solution**

| **Reagents** | **Amount** |
|---|---|
| Electrophoretic Premix | 9 µL |
| PCR amplified products | 1µL |

**TABLE 85. Electrophoresis Parameters**

| **Temperature (°C)** | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

5. Whether or not the sample to be tested contains the target nucleic acid is determined by comparing the fluorescent signal intensity difference of the nucleic acid probe that can be complementary to the detected nucleic acid and the nucleic acid probe that cannot be complementary to the detected nucleic acid. When the sample is negative, the number of fluorescent signals is consistent with the number of nucleic acid probes with fluorescent labels in the addition system, and the intensity of fluorescent signals at different positions is not significantly different; while when the sample is positive, the number of fluorescent signals is less than the number of nucleic acid probes with fluorescent labels in the addition system, or there is a significant difference in the intensity of fluorescent signals at different positions, and at this time, the disappearance fluorescent signal or the attenuated fluorescent signal represents that the position detection object is positive.

As shown in FIG. 17, when the fluorescent label of the nucleic acid probe is Cy5, the electrophoresis nucleic acid probe can be distinguished. When the detection sample is GAPDH, GAPDH corresponds to a significant decrease in the fluorescent signal peak of the nucleic acid probe. Compared with the prior art, in the prior art, whether the detection sample includes the detection target is determined by whether the fluorescent signal characteristic peak appears, and whether the detection sample includes the detection target is determined by whether the fluorescent signal characteristic peak is weakened.

### Example 18

This example is used to illustrate the effect of fluorescent signals when probes with different fluorescent labels are simultaneously electrophoretic.

The sequence of the nucleic acid probe used in this example is SEQ ID NO: 39, the fluorescent label used in SEQ ID NO. 40 is FAM, SEQ ID NO. 51, and the fluorescent label used in SEQ ID NO. 52 is VIC. The nucleic acid probes are synthesized by Shenggong bioengineering (Shanghai) Co. Ltd.
1. System Preparation:
Preparation system: Buffer (2 * qRT-PCR Premix [v7] (with UDG)) was mixed with a nucleic acid probe and water.

**TABLE 86 PCR SYSTEM**

| **Reagents** | **Amount (µL)** |
|---|---|
| 2*qRT-PCR Premix [v7] (with UDG) | 10 |
| Nucleic Acid Probe (200nM) | 1 |
| Water | 9 |
| Total Volume | 20 |

2. Capillary electrophoresis
The electrophoresis premix was formulated according to the formulation of Table 87.

**TABLE 87. Preparation of Electrophoretic Premix**

| **Reagents** | **Amount** |
|---|---|
| Hi-Di^{™} Formamide | 985 µL |
| SEQ ID NO. 59 (1µM) | 5 µL |
| SEQ ID NO. 60 (1µM) | 5 µL |
| SEQ ID NO. 61 (1µM) | 5 µL |

The PCR system was formulated according to the formulation of Table 88 using Applied Biosystems ^{™} 3500 Dx.

**TABLE 88. Preparation of Electrophoresis Solution**

| **Reagents** | **Amount** |
|---|---|
| Electrophoretic Premix | 9 µL |
| PCR system | 1µL |

**TABLE 89. Electrophoresis Parameters**

| **Temperature (°C)** | **Injection Voltage (v)** | **Injection Time (s)** | **Preoperation Voltage (v)** | **Preoperation Time (s)** | **Operation Voltage (v)** | **Operation Time (s)** |
|---|---|---|---|---|---|---|
| 60 | 1600 | 8 | 15000 | 180 | 19500 | 1200 |

3. As shown in FIG. 18, at the same time, probes with different fluorescent labels are electrophoretic, and the fluorescent signals do not interfere with each other in different detection channels. The FAM fluorescent signal appears only in the fluorescent channel 1, and the VIC fluorescent signal appears only in the fluorescent channel 2.

### SUMMARY OF THE COMPARISON WITH THE PRIOR ART

Based on the above summary and specific embodiments, the advantages of the capillary electrophoresis detection method of the present invention relative to the prior art detection method (FIG. 20) are summarized as follows with respect to the schematic diagram of the capillary electrophoresis detection method of the present invention (FIG. 19) and the detection method in the prior art (FIG. 20):
As shown in FIG. 20, in the prior art, the capillary electrophoresis (CE) detection technology has the following defects: 1) different targets are distinguished by separating different amplification products, so that the length of the amplification product is limited; 2) The amplification primer is used to carry the fluorescent label, and when the primer is initially non-specific amplified, the non-specific amplification product thereof affects the target signal interpretation; 3) the primer is used in excess, and when the concentration of the target nucleic acid sequence in the sample is too high, a saturated complex peak is generated, and the interference is determined adjacent to the target point.

As shown in FIG. 19, the present invention overcomes the above defects by adding a fluorescent label to the 5 ' end of the nucleic acid probe, so that the length of the amplification product is not limited by detecting different target points by detecting the nucleic acid probe; 2) the amplification system of the present invention only carries the fluorescent signal by using the nucleic acid probe, and no additional signal is generated; and 3) the present invention can effectively prevent the generation of the supersaturated fluorescent signal by using the specific concentration nucleic acid probe.

Although the above embodiments show the effects of some embodiments, it should be understood by those skilled in the art that, according to the concept of the present invention, the foregoing other embodiments that do not specifically show the effect, or other technical solutions of the present invention that are not shown in the embodiments or the examples, also achieve the following technical effects that are equivalent to the examples in the summary of the present invention:
1 in the present invention, different targets are identified by detecting the nucleic acid probe, so that the target point is not limited by the length of the amplification product;
2 in the amplification system of the present invention, only the nucleic acid probe carries the fluorescent signal and does not generate the non-specific fluorescent signal;
3 the present invention could effectively prevent the generation of supersaturated fluorescent signals by using specific concentration nucleic acid probes;
4 the fluorescent signals between different targets do not interfere with each other, and multi-color fluorescence can be used at the same time.

Furthermore, although this invention has been described to a certain extent, it will be apparent that suitable changes in various conditions may be made without departing from the spirit and scope of the invention. It is to be understood that the invention is not limited to the embodiments described, but is to be included within the scope of the claims, which include equivalents for each of the elements described.

## Claims

1. A primer/probe composition for capillary electrophoresis analysis, **characterized in that**, the primer/probe composition comprises:
a forward primer;
a reverse primer; and
a nucleic acid probe carrying a fluorescent label;
wherein neither the forward primer nor the reverse primer carries a fluorescent label, and the fluorescent label is preferably located at the 5' end of the nucleic acid probe.

2. The primer/probe composition according to claim 1, **characterized in that**, wherein:
the nucleic acid probe is a probe group with a nucleotide length difference of 1-100, and the nucleotide length difference of each nucleic acid probe in the probe group preferably is 1-50, more preferably is 1-20, and further preferably is 1-10;
the number of nucleotides of the forward primer sequence is 15-60, preferably is 18-40, more preferably is 18-30, and further preferably is 20-25;
the number of nucleotides of the reverse primer sequence is 15-60, preferably is 18-40, more preferably is 19-31, and further preferably is 19-26; and/or
the fluorescent label is one or more selected from the following: a fluorescent label with a maximum fluorescence emission wavelength of 400-525 nm, a fluorescent label with a maximum fluorescence emission wavelength of 526-560 nm, a fluorescent label with a maximum fluorescence emission wavelength of 561-580 nm, a fluorescent label with a maximum fluorescence emission wavelength of 581-620 nm, and a fluorescent label with a maximum fluorescence emission wavelength of 621-670 nm.

3. The primer/probe composition according to claim 2, **characterized in that**, wherein:
the fluorescent label with the maximum fluorescence emission wavelength at 400-525 nm is one or more selected from Alexa Fluor 405, Alexa Fluor 350, FAM, Alexa Fluor 488, and Atto 488, preferably is FAM or Alexa Fluor 488, most preferably is FAM;
the fluorescent label with the maximum fluorescence emission wavelength of 526-560 nm is one or more selected from JOE, TET, R6 G, VIC, HEX, and Alexa Fluor 532, preferably is one or more selected from JOE, VIC, and TET, more preferably is JOE or VIC;
the fluorescent label with the maximum fluorescence emission wavelength of 561-580 nm is one or more selected from Cy3, Alexa Fluor 555, NED, Alexa Fluor 546, and TAMRA, preferably is Cy3 or NED, and most preferably is Cy3;
the fluorescent label with the maximum fluorescence emission wavelength in 581-620 nm is one or more selected from ROX, Texas Red, Alexa Fluor 568, and Alexa Fluor 594, preferably is ROX or Texas Red, most preferably is ROX; and/or
the fluorescent label with the maximum fluorescence emission wavelength of 621-670 nm is one or more selected from Cy5, Alexa Fluor 633, and Alexa Fluor 647, preferably is Cy5 or Alexa Fluor 647, most preferably is Cy5.

4. The primer/probe composition according to any one of claims 1 to 3, **characterized in that**, wherein the nucleic acid probe is one or more selected from the following: a DNA probe, an RNA probe, a DNA/RNA chimeric probe, preferably is a DNA probe or an RNA probe, most preferably is a DNA probe; wherein:
the DNA probe is one or more selected from the following: a conventional DNA probe, a DNA probe comprising a lock nucleic acid, and a DNA probe comprising hypoxanthine, most preferably is a conventional DNA probe; and/or
the RNA probe is one or more selected from of the following: a conventional RNA probe, an RNA probe comprising a lock nucleic acid, and an RNA probe comprising hypoxanthine, most preferably is a conventional RNA probe.

5. The primer/probe composition according to any one of claims 1 to 4, **characterized in that**, wherein the 3' end of the nucleic acid probe further carries a label for inhibiting extension of the nucleic acid probe;
preferably, the label for inhibiting extension of the nucleic acid probe is one or more selected from the following: a fluorescent label, a phosphoric acid group, a non-complementary nucleotide, a ddNTP, and a Spacer modification, more preferably is one or more selected from the following: a phosphoric acid group, a non-complementary nucleotide, and a ddNTP, further preferably is a phosphoric acid group or a ddNTP.

6. An improved capillary electrophoresis detection method, **characterized in that**, the improved capillary electrophoresis detection method comprises the following steps:
1) preparing a reaction system using the primer/probe composition for capillary electrophoresis analysis according to any one of claims 1 to 5;
2) amplifying the reaction system prepared in step 1) by PCR reaction to obtain an amplification product;
3) separating the product amplified in step 2) by capillary electrophoresis.

7. The capillary electrophoresis detection method according to claim 6, **characterized in that**, wherein the step 1) comprises:
a) designing, synthesizing a forward primer and a reverse primer, and fluorescent labeling the nucleic acid probe;
b) preparing the synthesized forward primer and reverse primer, the fluorescent labeled nucleic acid probe, a reaction buffer, a DNA polymerase, dNTP, a sample to be tested, and a negative sample into a reaction system.

8. The capillary electrophoresis detection method according to claim 7, **characterized in that**, wherein in step b):
the negative sample is one or more selected from the following: purified water, a TE buffer, a plasmid sample that does not contain a target nucleic acid, a microbial isolate, and a tissue sample, preferably is one or more selected from the following: purified water, a TE buffer, a plasmid sample that does not contain a target nucleic acid, and a microbial isolate, more preferably is one or more selected from: purified water, a TE buffer, and a plasmid sample that does not contain a target nucleic acid; and/or
the reaction buffer is a Tris-hydrochloric acid buffer or a PBS buffer; and the reaction buffer preferably comprises a cation, and the cation is preferably one or more selected from magnesium ions, potassium ions, sodium ions, and calcium ions, more preferably is magnesium ions or potassium ions.

9. The capillary electrophoresis detection method according to claim 7 or 8, **characterized in that**, wherein in the reaction system formulated in step b):
the forward primer has a concentration of 50 nM -5000 nM, preferably 100 nM -2000 nM, more preferably 100 nM -300 nM;
the concentration of the reverse primer is 50 nM -5000 nM, preferably 100 nM -2000 nM, more preferably 100 nM -300 nM;
the concentration of the nucleic acid probe is 5 nM -500 nM, preferably 5 nM -200 nM, more preferably 5 nM -15 nM.

10. The capillary electrophoresis detection method according to any one of claims 7 to 9, **characterized in that**, wherein in step a):
the operation of fluorescent labeling the nucleic acid probe further includes one or more of the following:
(1) fluorescent labeling the 5' end of the nucleic acid probe while modifying the 3' end of the nucleic acid probe,
(2) fluorescent labeling the 5' end of the nucleic acid probe while making the 3' end base of the nucleic acid probe not completely complementary to the template, and
(3) fluorescent labeling the 3' end of the nucleic acid probe.

11. The capillary electrophoresis detection method according to any one of claims 6 to 10, **characterized in that**, wherein the improved capillary electrophoresis detection method further comprises:
4) analyzing the target nucleic acid in the sample to be tested by comparing the fluorescent signal intensity of the nucleic acid probe.

12. The use of the primer/probe composition according to any one of claims 1 to 5 in the manufacture of a reagent product and/or detection device for capillary electrophoresis analysis, wherein:
the reagent product is preferably a kit; and/or
the detection device is preferably a capillary electrophoresis instrument or a DNA sequencer;
preferably, the DNA sequencer is a Sanger sequencer.

13. A reagent product for capillary electrophoresis analysis, **characterized in that**, wherein the reagent product comprises the primer/probe composition according to any one of claims 1 to 5;
preferably, the reagent product is a kit.

14. A detection device for capillary electrophoresis analysis, **characterized in that**, wherein the detection device comprises the primer/probe composition according to any one of claims 1 to 5.

15. The detection device according to claim 14, **characterized in that**, wherein the detection device is a capillary electrophoresis instrument or a DNA sequencer;
Preferably, the DNA sequencer is a Sanger sequencer.
